# EUROPEAN PATENT SPECIFICATION

(11) **EP 2 152 293 B1**
(45) Date of publication and mention of the grant of the patent: **19.10.2016**
(21) Application number: 08780622.0
(22) Date of filing: 09.05.2008
(51) Int. Cl.: A61K 38/16, A61K 38/28

(54) **COMPOSITIONS FOR PROTEIN DELIVERY AND METHODS OF USE THEREOF**
ZUSAMMENSETZUNGEN ZUR PROTEINABGABE UND VERFAHREN ZU DEREN VERWENDUNG
COMPOSITIONS DE LIVRAISON DE PROTÉINES ET PROCÉDÉS D'UTILISATION DE CES DERNIÈRES

(30) Priority: 11.05.2007 US 928884 P; 05.12.2007 US 5463 P
(43) Date of publication of application: 17.02.2010
(73) Proprietor: The Board of Regents of the University of Nebraska, Omaha, NE 68198-6099 (US)
(72) Inventor: KABANOV, Alexander, V., Omaha, NE 68154 (US); BRONICH, Tatiana, Omaha, NE 68135 (US); BATRAKOVA, Elena, Omaha, NE 68164 (US); GENDELMAN, Howard, Omaha, NE 68154 (US)
(74) Representative: Cripps, Joanna Elizabeth
(86) International application number: PCT/US2008/063213
(87) International publication number: WO 2008/141155

(56) References cited:
- H. DOU ET AL: "Development of a macrophage-based nanoparticle platform for antiretroviral drug delivery", BLOOD, vol. 108, no. 8, 15 October 2006 (2006-10-15), pages 2827-2835, XP055047636, ISSN: 0006-4971, DOI: 10.1182/blood-2006-03-012534
- DOU ET AL: "Laboratory investigations for the morphologic, pharmacokinetic, and anti-retroviral properties of indinavir nanoparticles in human monocyte-derived macrophages", VIROLOGY, ACADEMIC PRESS,ORLANDO, US, vol. 358, no. 1, 9 January 2007 (2007-01-09), pages 148-158, XP005762425, ISSN: 0042-6822, DOI: 10.1016/J.VIROL.2006.08.012
- VINOGRADOV S V ET AL: "Nanosized Cationic Hydrogels for Drug Delivery: Preparation, Properties and Interaction with Cells", ADVANCED DRUG DELIVERY REVIEWS, ELSEVIER BV, AMSTERDAM, NL, vol. 54, no. 1, 1 January 2002 (2002-01-01), pages 135-147, XP008123034, ISSN: 0169-409X
- HARADA A ET AL: "Pronounced activity of enzymes through the incorporation into the core of polyion complex micelles made from charged block copolymers", JOURNAL OF CONTROLLED RELEASE, ELSEVIER, AMSTERDAM, NL, vol. 72, no. 1-3, 14 May 2001 (2001-05-14) , pages 85-91, XP027296120, ISSN: 0168-3659 [retrieved on 2001-05-14]
- ATSUSHI HARADA ET AL: "Switching by Pulse Electric Field of the Elevated Enzymatic Reaction in the Core of Polyion Complex Micelles", JOURNAL OF THE AMERICAN CHEMICAL SOCIETY, vol. 125, no. 50, 1 December 2003 (2003-12-01), pages 15306-15307, XP055047768, ISSN: 0002-7863, DOI: 10.1021/ja038572h
- ELENA V. BATRAKOVA ET AL: "A Macrophage-Nanozyme Delivery System for Parkinson's Disease", BIOCONJUGATE CHEMISTRY, vol. 18, no. 5, 1 September 2007 (2007-09-01), pages 1498-1506, XP055047294, ISSN: 1043-1802, DOI: 10.1021/bc700184b
- NATALIA L KLYACHKO ET AL: "Cross-linked antioxidant nanozymes for improved delivery to CNS", NANOMEDICINE: NANOTECHNOLOGY, BIOLOGY AND MEDICINE, ELSEVIER, NL, vol. 8, no. 1, 20 May 2011 (2011-05-20), pages 119-129, XP028122792, ISSN: 1549-9634, DOI: 10.1016/J.NANO.2011.05.010 [retrieved on 2011-06-03]
- VINOGRADOV S.V. ET AL.: 'Nanosized Cationic Hydrogels for Drug Delivery: Preparation, Properties and Interaction with Cells' ADVANCED DRUG DELIVERY REVIEWS vol. 54, no. 1, January 2002, pages 135 - 147, XP008123034
- WU J. ET AL.: 'Quantitative Evaluation of Monocyte Transmigration into the Brain Following Chemical Opening of the Blood-Brain Barrier in Mice' BRAIN RESEARCH vol. 1098, no. 1, July 2006, pages 79 - 85, XP025065108
- BALLABH P. ET AL.: 'The Blood-Brain Barrier: An Overview Structure, Regulation, and Clinical Implications' NEUROBIOLOGY OF DISEASE vol. 16, no. 1, June 2004, pages 1 - 13, XP008123035
- KAKIZAWA Y. ET AL.: 'Block copolymer micelles for delivery of gene and related compounds' ADVANCED DRUG DELIVERY vol. 54, no. 2, February 2002, pages 203 - 222, XP001150717
- VINOGRADOV S.V. ET AL.: 'Polyion Complex Micelles with Protein-Modified Corona for Receptor-Mediated Delivery of Oligonucleotides into Cells' BIOCONJUGATE JOURNAL vol. 10, no. 5, September 1999, pages 851 - 860, XP000854010
- VINOGRADOV S. ET AL.: 'Self-Assembly of Polyamine-Poly(ethylene glycol) Copolymers with Phosphorothioate Oligonucleotides' BIOCONJUGATE JOURNAL vol. 9, no. 6, November 1998, pages 805 - 812, XP002521358
- AKTAS Y. ET AL.: 'Development and Brain Delivery of Chitosan-PEG Nanoparticles Functionalized with the Monoclonal Antibody OX26' BIOCONJUGATE JOURNAL vol. 16, no. 6, November 2005, pages 1503 - 1511, XP008123076

## Description

### FIELD OF THE INVENTION

The present invention relates to compositions and methods for the delivery of therapeutic agents to a patient, particularly to the central nervous system (CNS).

### BACKGROUND OF THE INVENTION

The blood-brain barrier (BBB) is one of the most restrictive barriers in biology. Numerous factors work together to create this restrictive barrier. Electron microscopy studies have demonstrated that tight junctions between brain vascular endothelial cells and other endothelial cell modifications (e.g., decreased pinocytosis, lack of intracellular fenestrae) prevented the formation of a plasma ultrafiltrate. Enzymatic activity at the BBB further limits entry of some substances, especially of monoamines and some small peptides (Baranczyk-Kuzma and Audus (1987) J. Cereb. Blood Flow Metab., 7:801-805; Hardebo and Owman (1990) Pathophysiology of the BBB, pp. 41-55 (Johansson et al., Eds.) Elsevier, Amsterdam; Miller et al. (1994) J. Cell. Physiol., 161:333-341; Brownson et al. (1994) J. Pharmacol. Exp. Ther., 270:675-680; Brownlees and Williams (1993) J. Neurochem., 60:793-803). Saturable, brain-to-blood efflux systems, such as p-glycoprotein (Pgp), also prevent the accumulation of small molecules and lipid soluble substances (Taylor, E.M. (2002) Clin. Pharmacokinet., 41:81-92; Schinkel et al. (1996) J. Clin. Invest., 97:2517-2524). Peripheral factors such as protein binding/soluble receptors, enzymatic degradation, clearance, and sequestration by tissues also affect the ability of a substance to cross the BBB by limiting presentation; these factors are especially important for exogenously administered substances (Banks and Kastin (1993) Proceedings of the International Symposium on Blood Binding and Drug Transfer, pp. 223-242 (Tillement et al., Eds.) Fort and Clair, Paris). H. Dou et al (BLOOD, vol. 108; 15 October 2006, pages 2827-2835) describes the development of a macrophage-based nanoparticle platform for antiretroviral drug delivery, Dou et al (Virology, Academic press, Orlando US, vol. 358, no. 1, 9 January 2007) investigates the morphologic, pharmacokinetic, and anti-retroviral properties of indinavir nanoparticles in human monocyte-derived macrophages.

Vinogradov et al (Advanced Drug Delivery Reviews 54 (2002) 135-147) looks at nanosized cationic hydrogels for drug deliver and discusses their properties and interaction with cells. They conclude that the delivery system has a potential of enhancing oral and brain bioavailability of oligonucleotides.

Harada et al (J. Controlled Release, Vol. 72, no. 1-3, p. 85-91; 14 May 2001) study the enzymatic activity of lysozyme in the core of polyion complex (PIC) micelles which were formed from egg white lysozyme and poly(ethylene glycol)-poly(α, β-aspartic acid) block copolymer (PEG-P(Asp)). The results indicate that the enzymatic activity can be controlled by changing the corona thickness of the PIC micelles through a variation in then mixing ratio of PEG-P(Asp) to lysozyme.

Harada et al (J. American Chem Soc, vol. 125, no. 50, 1 December 2003) further report on the elevation of the lysozyme activity through the inclusion into the PIC micelles which achieves the switching of enzyme reactivity synchronising with the application of a pulse electric field.

### SUMMARY OF THE INVENTION

In accordance with the instant invention, pharmaceuticals compositions for use in treating a neurological disorder of the central nervous system in a patient are provided.

More specifically, there is provided a pharmaceutical composition for use in treating a neurological disorder of the central nervous system, said composition comprising:
a) at least one complex comprising a therapeutic polypeptide and a synthetic polymer, wherein said synthetic polymer is a block copolymer comprising at least one water soluble, nonionic segment and at least one polyion segment, wherein said complex self-assembles into nanoparticles having a core-shell morphology wherein the core comprises the therapeutic polypeptide and the polyion segment of the block copolymer, and wherein said polyion segment comprises at least one charge opposite to the charge of the therapeutic polypeptide, and
b) at least one pharmaceutically acceptable carrier.

In some embodiments the complex may be comprised within an isolated cell and the cell may be isolated from the patient to be treated. The isolated cell may be an immune cell. For example, the immune cell may be selected from the group consisting of bone marrow monocytes, monocytes, macrophages, bone marrow derived monocytes, dendritic cells, lymphocytes, T-cells, neutrophils, eosinophils, and basophils.

### BRIEF DESCRIPTIONS OF THE DRAWING

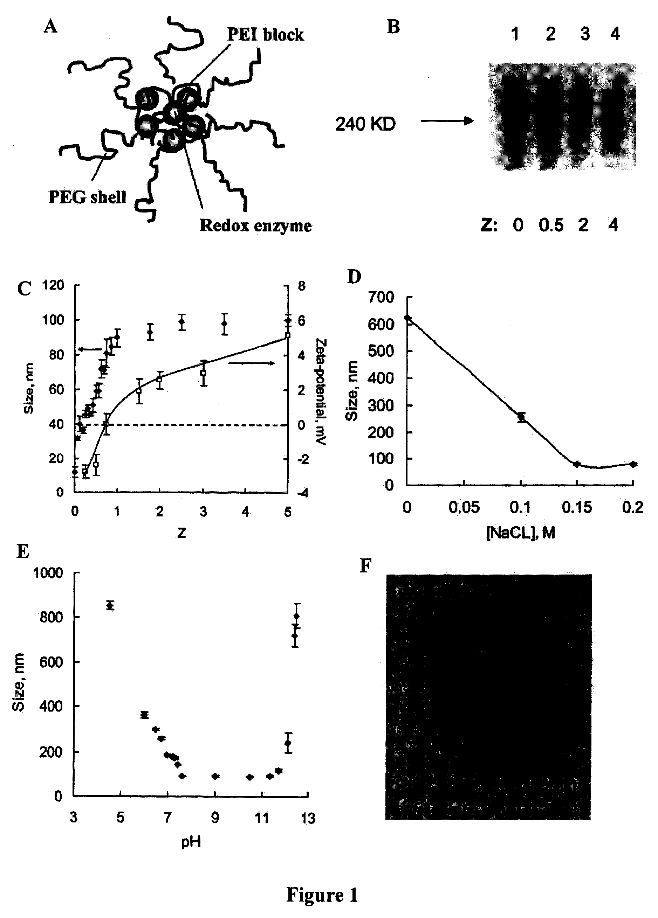

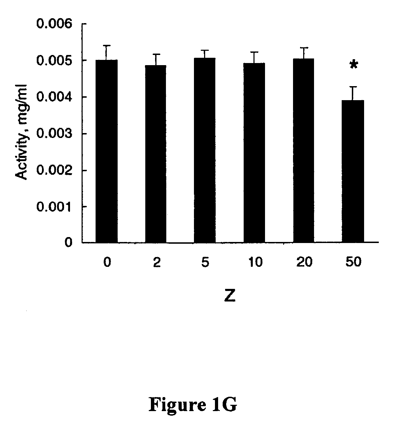

Figure 1A provides a schematic presentation of a polypeptide-polyion complex structure (may also be referred to as a nanozyme). Figure 1B is an image of a gel retardation assay of the enzyme/polyion complexes at various Z. Samples were subjected to gel electrophoresis in polyacrylamide gel (7.5%) under nondenaturing conditions (without SDS). Lane 1: enzyme alone; lanes 2-4: enzyme/PEI-PEG complexes with progressive increasing of Z (0.5, 2, 4). Figures 1C-E are graphs of the changes in cumulant diameter (Figs 1C-E) and zeta-potential (Fig. 1C) of catalase-polyion complexes under various conditions: Figure 1C: Z in PBS solutions; Figure 1D: ionic strength (Z = 1, pH 7.4); Figure 1E: pH (Z = 1, [NaCl] = 0.15M). Figure 1F is a TEM image of catalase-polyion complex (Z = 1). Bar represents 100 nm. Figure 1G is a graph of the enzymatic activity of catalase in polyion complex. The activity of catalase in polyion complex with various Z was determined by the rate of hydrogen peroxide decomposition. Data represent means ± SEM (n=4). Statistical significance of catalase-polyion complex activity compared to catalase alone is shown by asterisks: (*) p<0.05. The enzymatic activity of catalase was not changed over wide range of the block copolymer, significantly decreasing only at Z=50.

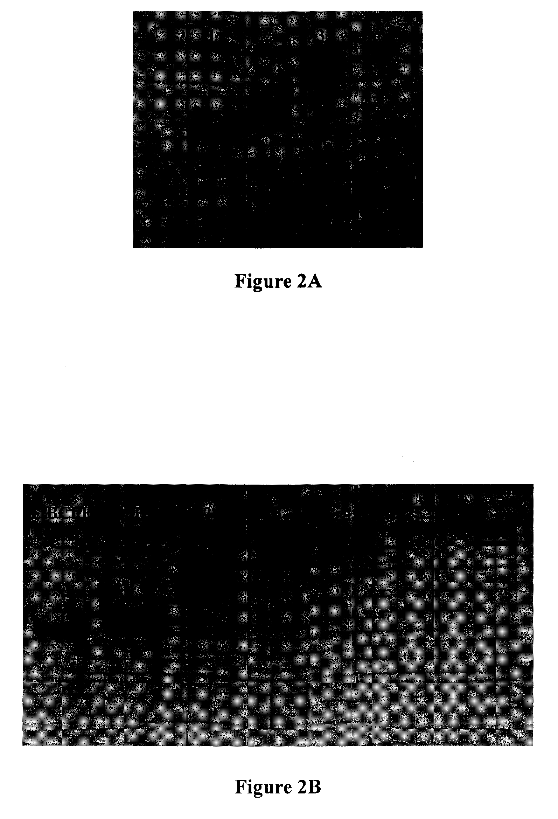

Figure 2A is an image of a gel electrophoresis assay of Hu BChE/PLL-g-PEO(2) complexes. Lane numbers correspond to the sample numbers in Table 1. Figure 2B is an image of a gel electrophoresis assay of Hor BChE alone and Hor BChE/PLL-g-PEO(2) complexes at various compositions. Lane numbers correspond to the sample numbers in Table 2.

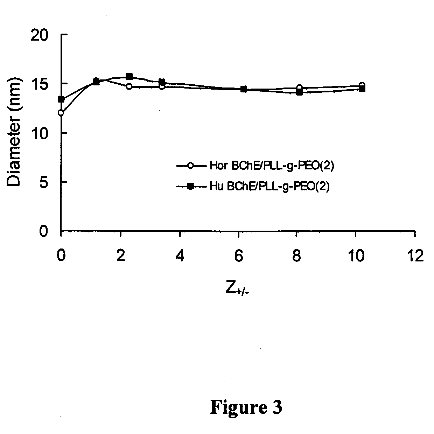

Figure 3 is a graph of the diameter of the particles formed in (o) Hor BChE/PLL-g-PEO(2) and (■) Hu BChE/PLL-g-PEO(2) mixtures at various Z+/-. Concentration of BChE was 0.15 mg/ml, 23°C, 10 mM phosphate buffer, pH 7.4.

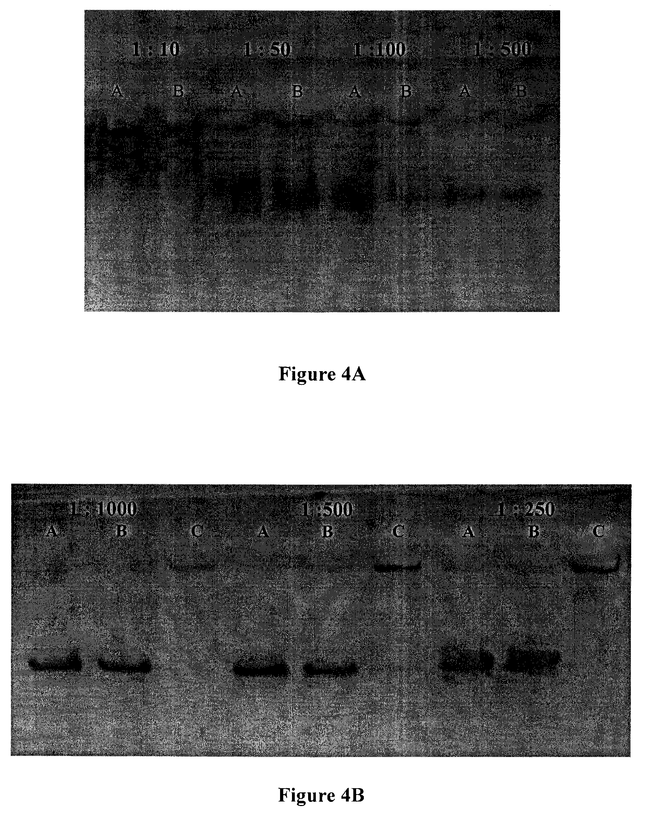

Figure 4A is an image of a gel electrophoresis assay of Hor BChE alone (A) and Hor BChE/ PLL-g-PEO(7) complex (B) (Z_{+/-}= 10.3) at various dilutions. The initial concentration of Hor BChE was 0.167 mg/ml. Figure 4B is an image of a gel electrophoresis assay of Hu BChE alone (A); non cross-linked Hu BChE/ PLL-g-PEO(2) complexes (B) (Z_{+/-}= 1.2); and cross-linked Hu BChE/ PLL-g-PEO(2) complexes (C) (Z_{+/-}= 1.2; 85% cross-linking ratio), at various dilutions (1:1000, 1:5000, and 1:250). The initial concentration of Hu BChE was 0.15 mg/ml.

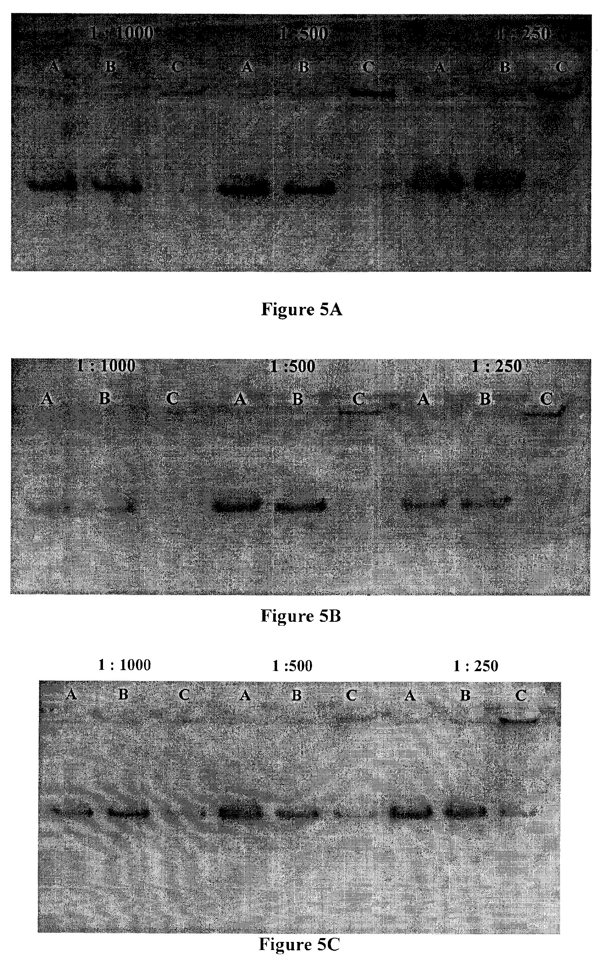

Figures 5A-5C provide images of gel electrophoresis assays of Hu BChE alone (lane A); non cross-linked Hu BChE/ PLL-g-PEO(2) complexes (lane B) (Z_{+/-}= 1.2); and cross-linked Hu BChE/ PLL-g-PEO(2) complexes (lane C) (Z_{+/-}= 1.2) at various dilutions: 1000, 500, and 250. The cross-linking ratio was 85%, 40%, and 20% in Figures 5A, 5B, and 5C, respectively. The final concentration of Hu BChE was 0.15 mg/ml.

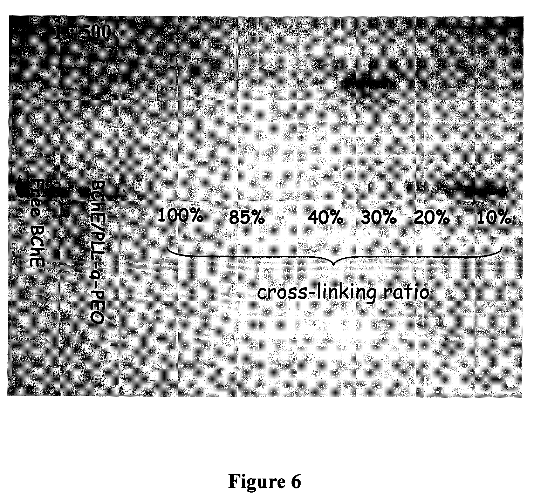

Figure 6 is an image of a gel electrophoresis assay of cross-linked Hu BChE/PLL-g-PEO(2) complexes (Z+/-= 1.2) of various cross-linking ratio, at 500-fold dilution. The final concentration of Hu BChE was 0.15 mg/ml.

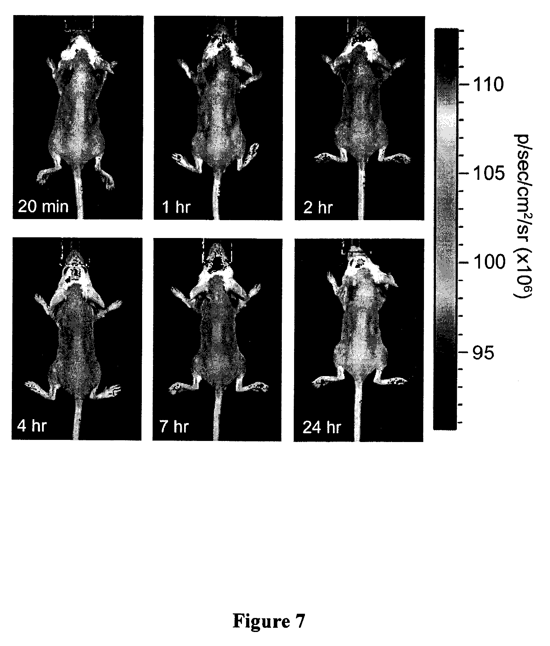

Figure 7 provides images of mice intravenously injected with CuZnSOD-polyion complex. Using an IVIS 200 imaging system, Alexa 680 fluorescence was detected in mice at various time intervals following intravenous (tail vein) injection of Alexa 680-labeled CuZnSOD-polyion complex.

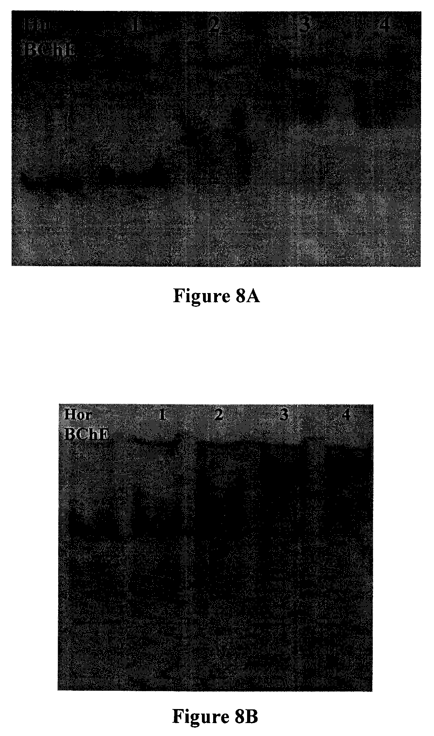

Figures 8A and 8B provide images of gel electrophoresis assays of Hu BChE/PLL-b-PEO complexes and Hor BChE/PLL-b-PEO complexes, respectively. The lane numbers correspond to the sample numbers provided in Table 9. The concentration of Hu BChE and Hor BChE was 0.15 mg/ml.

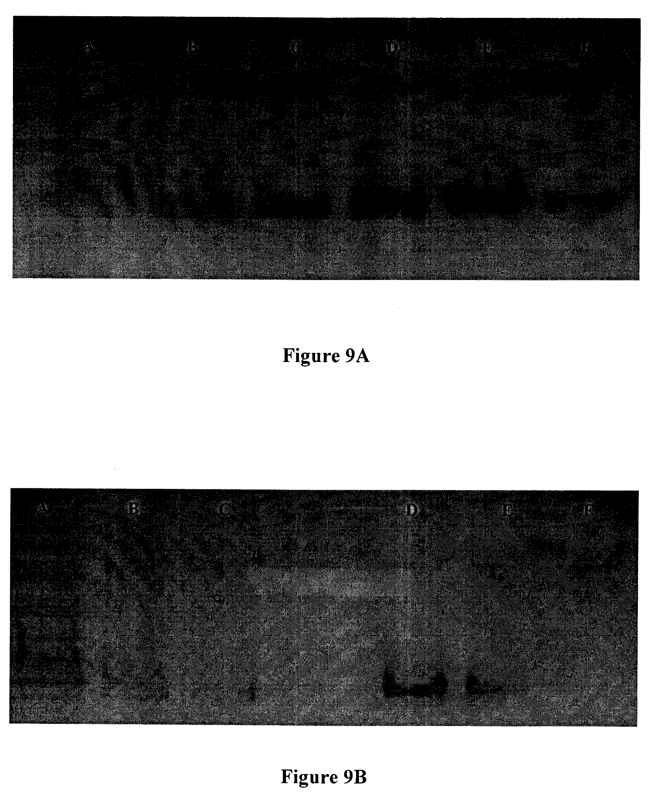

Figures 9A and 9B is an image of a gel electrophoresis assay of cross-linked Hu BChE/PLL-b-PEO and Hor BChE/PLL-b-PEO complexes at Z+/-= 1.0 or at Z+/-= 2.0, respectively, with a 40% cross-linking ratio. Lane A is Hu BChE alone; lane B is non cross-linked Hu BChE/PLL-b-PEO complex; lane C is cross-linked Hu BChE/PLL-b-PEO complex; lane D is Hor BChE alone; lane E is non cross-linked Hor BChE/PLL-b-PEO complex; and lane F is cross-linked Hor BChE/PLL-b-PEO complex. The final concentration of BChE was 0.0003 mg/ml.

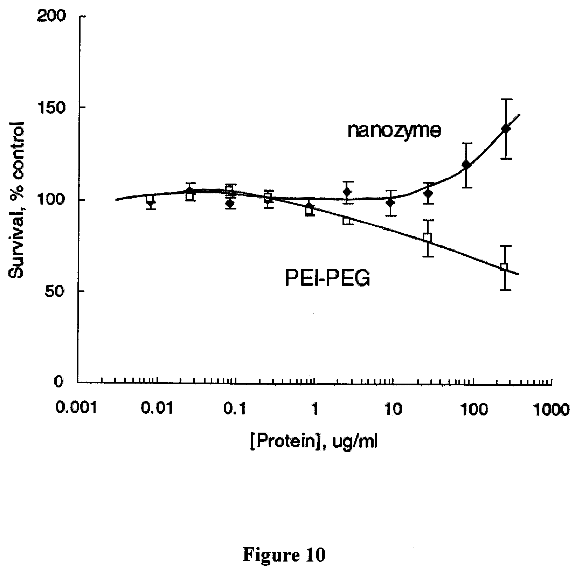

Figure 10 is a graph the cytotoxicity of polypeptide-polyion complex (Z=1) or the corresponding concentrations of PEI-PEG in BMM. Cells were incubated for 24 hours with various concentrations of polypeptide-polyion complex or the block copolymer, washed, and incubated in the fresh media for 48 hours at 37°C. Cell survival was determined by sulforhodamine-B (SRB) assay. Absorbance was measured at 490 nm in Microkinetics reader BT2000 and obtained values were expressed as a percentage of the values obtained for control cells to which no polypeptide-polyion complexes were added. All measurements were repeated eight times. No cytotoxic effects of catalase alone or polyion complex of catalase and PEI-PEG in BMM were observed.

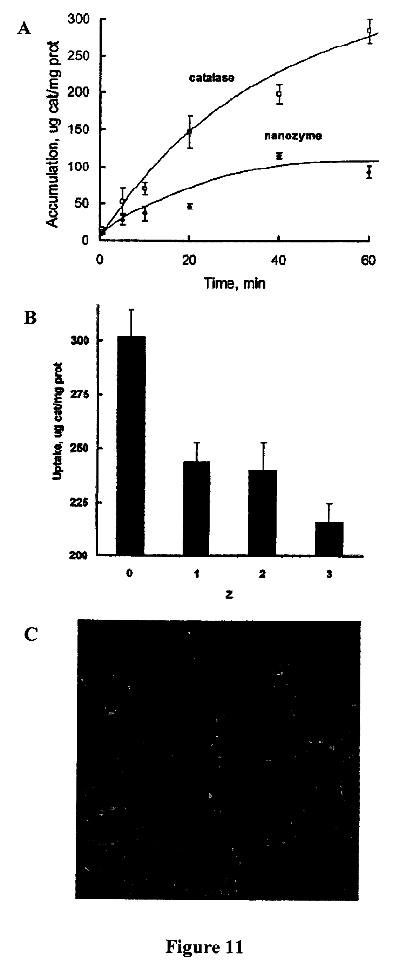

Figure 11A is a graph of the kinetics of "naked" catalase and catalase-polyion complex (Z = 1) accumulation in monocytes. Cells were treated with the Alexa Fluor 594 labeled enzyme or enzyme-polyion complex at various time points. Following incubation, the cellular content was collected, and the amount of fluorescence was measured by fluorescent spectrophotometer (λₑₓ = 580 nm, λₑₘ = 617 nm). Data represent means ± SEM (n = 4). Figure 11B is a bar graph depicting the accumulation of catalase-polyion complexes in BMM at various Z. Figure 11C provides an image of the intracellular localization of RITC-labeled catalase-polyion complex in BMM. Cells grown on cover slips were loaded with catalase/PEI-PEG complex (Z = 1) for 24 hours. Following the incubation, the cells were fixed and stained with F-actin-specific Oregon Green 488 phalloidin and a nuclear stain, ToPro-3. Images were obtained by confocal fluorescence microscopic system ACAS-570.

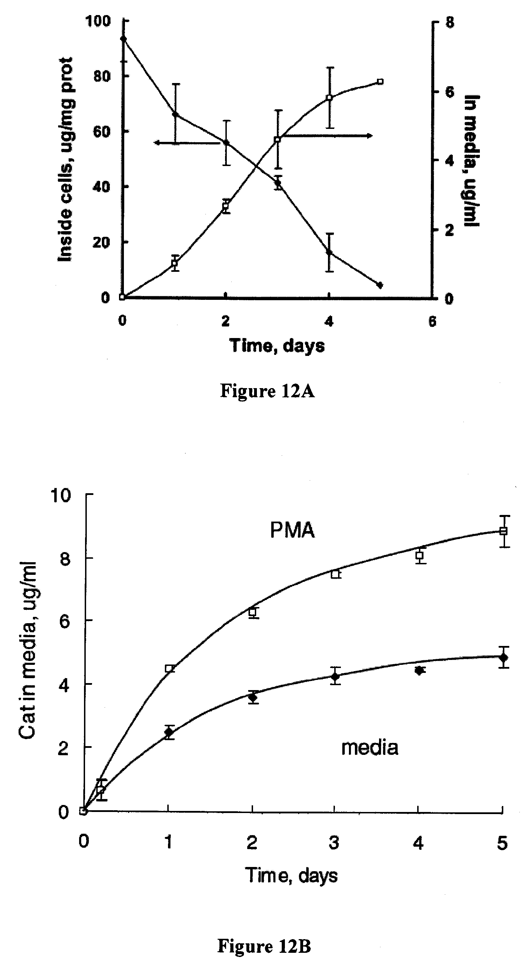

Figure 12A is a graph of the release profile of catalase-polyion complex from BMM. Cells were loaded with catalase/PEI-PEG complex (Z = 1) for 1 hour, washed with PBS, and incubated with catalase-free media for various time intervals. Amount of catalase released into the media and retained in the cells was accounted by fluorescent spectrophotometry. Data represent means ± SEM (n = 4). Figure 12B is a graph of the triggered release of catalase from BMM in the media. Mature BMM were pre-loaded with Alexa Fluor 594-labeled catalase-polyion complex (Z = 1) for 1 hour, washed with PBS, and then incubated catalase-free media with or without 10 µM phorbol myristate acetate (PMA) for various time intervals. The amount of catalase released into the media was accounted by fluorescent spectrophotometry. Data represent means ± SEM (n = 4). Addition of PMA to the incubation media resulted in the enhanced the enzyme release in the media by ca. 50%.

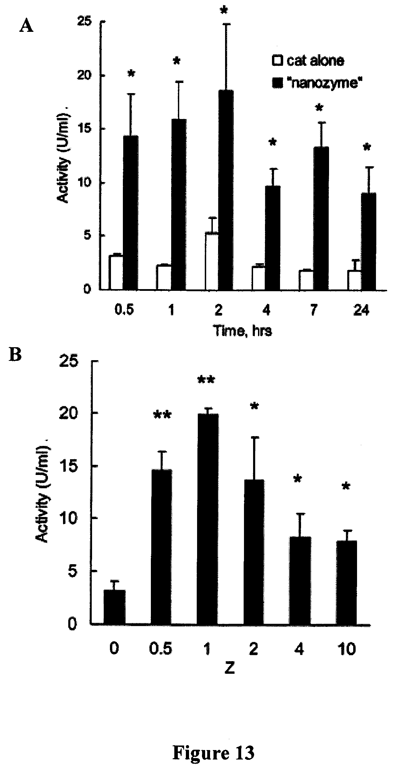

Figures 13A and 13B are graphs depicting the preservation of enzymatic activity of catalase against degradation in BMM. In Figure 13A, "naked" catalase or catalase-polyion complex (Z = 1) were loaded into BMM, and cells were washed and incubated with catalase-free media for various time intervals. The activity of catalase released from BMM was determined by spectrophotometry. In Figure 13B, catalase polyion complexes with various compositions (Z) were loaded into the cells and incubated in catalase-free media for 2 hours. Then, the media was collected and assessed for catalase activity by spectrophotometry. Data represent means ± SEM (n = 4). Statistical significance of catalase-polyion complex activity compared to catalase alone is shown by asterisks: (*) p < 0.05, (**) p < 0.005.

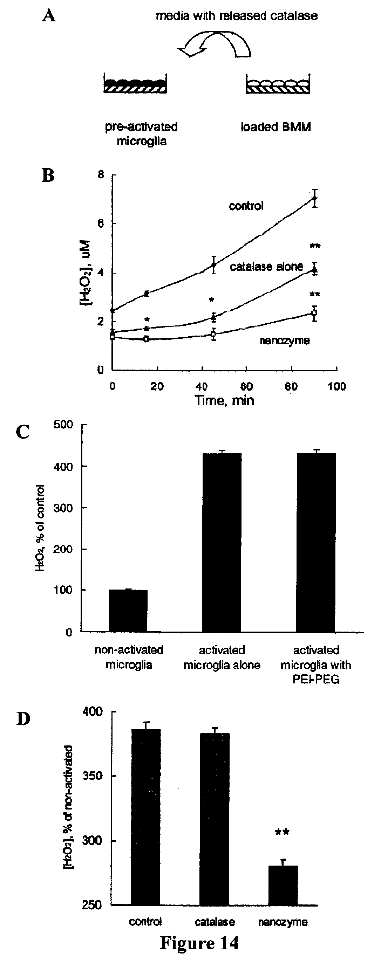

Figure 14A is a scheme for the modulation of microglial-derived ROS by catalase-polyion complex released from BMM. Block copolymer (2 mg/ml; Figure 14C) or "Naked" catalase or catalase-polyion complex (Z = 1) (Figures 14B and 14D) were loaded into BMM. Then, cells were washed and incubated in Kreb's Ringer buffer for 2 hours. In parallel, murine microglial cells were either stimulated with 200 ng/mL TNF-α (48 hours) (Figs. 14B and 14C) or 0.5 µM N-α-syn (Fig. 14D). Then, supernatants collected from BMM with the released enzyme were supplemented with Amplex Red and HRP solutions and added to the activated microglial cells. Control activated microglia was incubated with fresh media (Fig. 14B) or 0.5 µM aggregated N-α-syn (Fig. 14D). The amount of H₂O₂ produced by microglial cells and decomposed by catalase released from BMM was detected by fluorescence. Data represent mean ± SEM (n = 6). Statistical significance of the amount of H₂O₂ decomposed by released from BMM catalase-polyion complex or catalase, compared to activated microglia (control) is shown by asterisks: (*) p < 0.05, (**) p < 0.005.

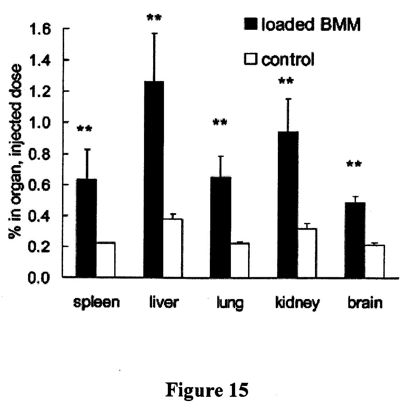

Figure 15 is a graph of the biodistribution of **¹²⁵I-**labeled catalase-polyion complex in MPTP-treated mice. Mice were injected with BMM (10 x 10⁶ cells/mouse) loaded with catalase-polyion complex (Z = 1, 50 µCi/mouse) or with catalase-polyion complex alone (control group). Twenty-four hours later mice were sacrificed and the amount of radioactivity was measured in various organs. Data represent mean ± SEM (n = 4). Statistical significance of the BMM-loaded catalase-polyion complex transport compared to the catalase-polyion complex alone group is shown by asterisks: (**) p < 0.005.

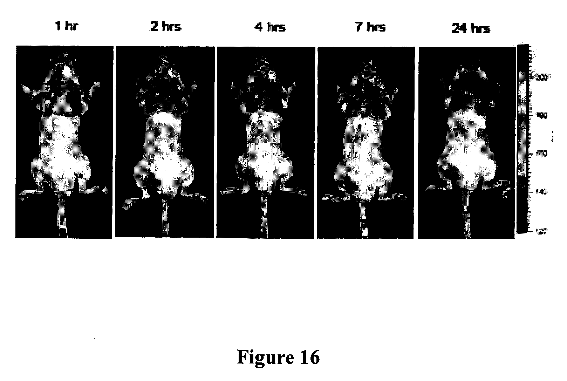

Figure 16 provides images of the biodistibution over time of Alexa 680-labeled polypeptide-polyion complex loaded to BMM and injected intravenously to MPTP-intoxicated mice.

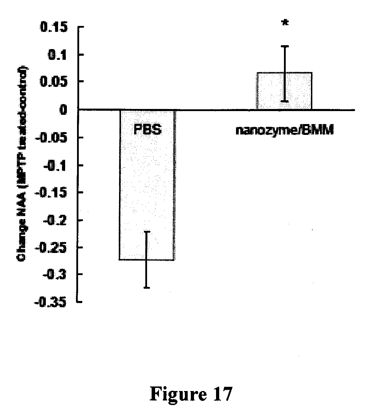

Figure 17 is a graph demonstrating neuroprotection against MPTP-induced dopaminergic neuronal loss by the administration of BMM comprising a polypeptide-polyion complex loaded with catalase. A significant decrease in NAA levels was observed in control mice with a slight increase in catalase-polyion complex/BMM treated mice (n=4).

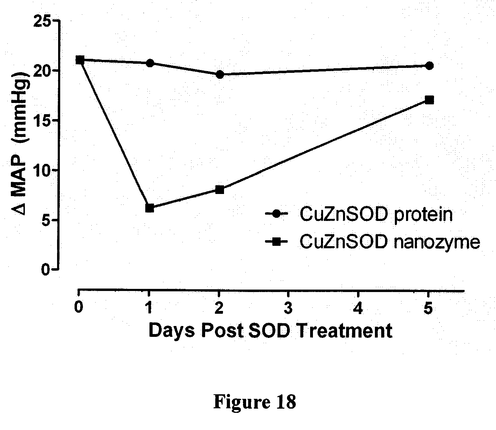

Figure 18 is a graph demonstrating CuZnSOD-polyion complex peripherally administered inhibits ICV AngII-mediated increase in blood pressure. Peak change in mean arterial pressure (MAP) following ICV-injected AngII was measured 0, 1, 2, and 5 days after intra-carotid administration of free CuZnSOD or CuZnSOD-polyion complex.

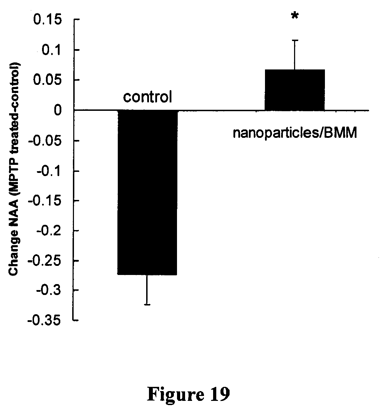

Figure 19 is a graph depicting neuroprotection against MPTP-induced dopamineegic neuronal loss with BMM loaded with a catalase polyion complex.

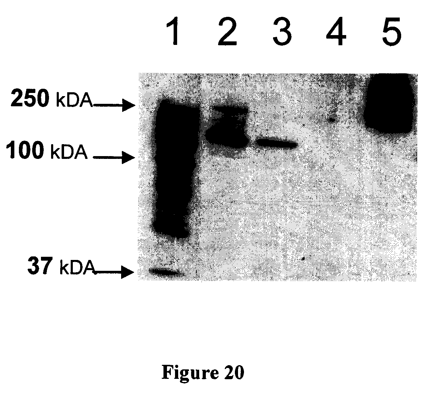

Figure 20 is an image of a gel retardation assay of the catalase/polyion complexes with various cross-linkers used. Samples were subjected to gel electrophoresis in polyacrylamide gel (10 %) under denaturing conditions (with SDS). Lanes: 1- molecular weight markers; 2- catalase alone; and polyion complexes linked with 3-EDC; 4-GA; 5-BS3.

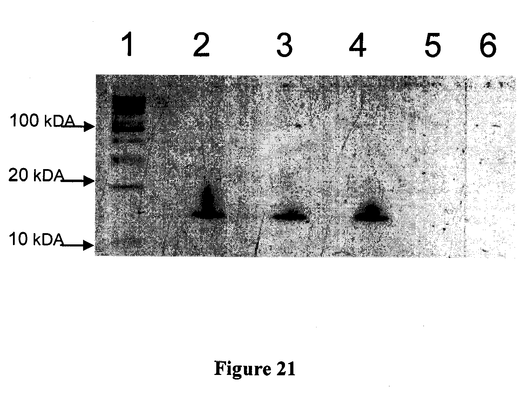

Figure 21 is an image of a gel retardation assay of the SOD/polyion complexes for various linkers used. Samples were subjected to gel electrophoresis in polyacrylamide gel (10 %) under denaturing conditions (with SDS). Lanes: 1- molecular weight markers; 2-SOD alone; 3- non-linked polyion complex; and polyion complexes linked with 4-EDC; 5-GA; 6-BS3.

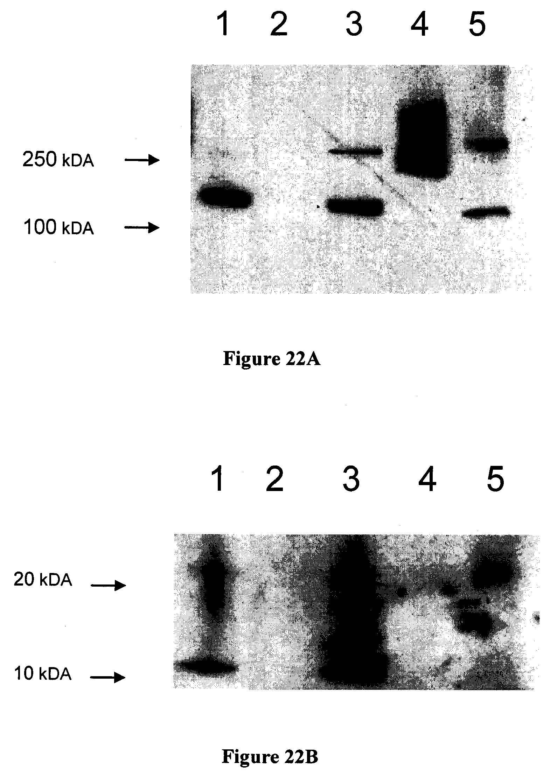

Figure 22A is an image of a gel retardation assay of the catalase/SOD/polyion complexes for various linkers used. Samples were subjected to gel electrophoresis in polyacrylamide gel (10 %) under denaturing conditions (with SDS). Lanes: 1-non-linked complex; polyion complexes linked with 2-GA; 3-EDC; 4-BS3; and 5-EDC-S-NHS. Visualization was performed with antibody to catalase. Figure 22B is an image of a gel retardation assay of the catalase/SOD/polyion complexes for various linkers used. Samples were subjected to gel electrophoresis in polyacrylamide gel (10 %) under denaturing conditions (with SDS). Lanes: 1-non-linked complex; polyion complexes linked with 2-GA; 3-EDC; 4-BS3; and 5-EDC-S-NHS. Visualization was performed with antibody to SOD.

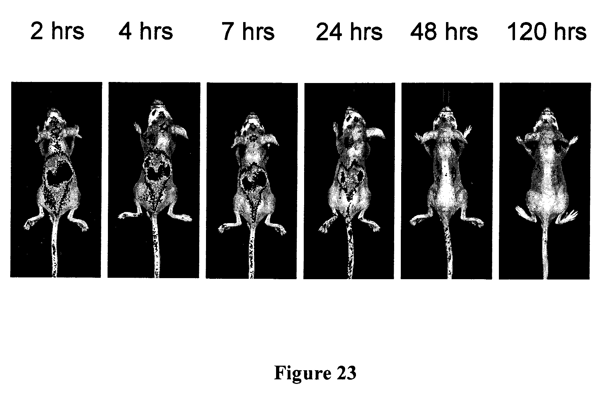

Figure 23 provides images of the biodistribution of Li-COR-labeled BMM loaded with catalase polyion complex. BMM were isolated from BALB/C mice, grown till maturation (12 days) labeled with Li-COR, and loaded for 2 hours with catalase polyion complex. Loaded BMM were injected i.v. into shaved BALB/C (50 mln/mouse) kept on liquid diet for 24 hours.

### DETAILED DESCRIPTION OF THE INVENTION

In accordance with the present invention, compositions are provided for the site-specific and/or sustained delivery of a protein/polypeptide of interest. More specifically, the compositions comprise a polyion complex of the polypeptide of interest with a synthetic polymer having a net charge opposite to the net charge of the protein of interest.

In a preferred embodiment of the instant invention, the synthetic polymers of the complexes are block copolymers. More specifically, the synthetic polymers are block copolymers which comprise at least one polyion segment and at least one nonionic water soluble polymer segment. Block copolymers are most simply defined as conjugates of at least two different polymer segments (Tirrel, M. In: Interactions of Surfactants with Polymers and Proteins. Goddard E.D. and Ananthapadmanabhan, K.P. (eds.), CRC Press, Boca Raton, Ann Arbor, London, Tokyo, pp. 59-122, 1992). The simplest block copolymer architecture contains two segments joined at their termini to give an A-B type diblock. Consequent conjugation of more than two segments by their termini yields A-B-A type triblock, A-B-A-B-type multiblock, or even multisegment A-B-C-architectures. If a main chain in the block copolymer can be defined in which one or several repeating units are linked to different polymer segments, then the copolymer has a graft architecture of, e.g., an A(B)ₙ type. More complex architectures include for example (AB)ₙ or AₙBₘ starblocks which have more than two polymer segments linked to a single center. An exemplary block copolymer of the instant invention would have the formula A-B or B-A, wherein A is a polyion segment and B is a nonionic water soluble polymer segment. The segments of the block copolymer may have from about 2 to about 1000 repeating units or monomers.

The preferred size of the complexes is between about 5 nm and about 500 nm, more preferred between about 5 and about 250 nm, more preferred between about 10 and about 150 nm, still more preferred between about 10 nm and about 140 nm, yet still more preferred between about 20 and about 100 nm. The complexes do not aggregate and remain within the preferred size range for at least 1 hour after dispersion in the aqueous solution at the physiological pH and ionic strength, for example in phosphate buffered saline, pH 7.4. The sizes may be measured as effective diameters by dynamic light scattering (see, e.g., Batrakova et al. (2007) Bioconjugate Chem., 18:1498-1506). It is preferred that, after dispersion in aqueous solution, the complexes remain stable, i.e., do not aggregate and/or precipitate for at least 2 hours, preferably for 12 hours, still more preferably for 24 hours.

The polyion segment of the block copolymer has a net charge which is opposite to the protein of interest. For example, if the protein of interest has a net negative charge, then the polyion segment will have a net positive charge, at the relevant pH. The polyion segment may be a polycation (i.e., a polymer that has a net positive charge at a specific pH) or a polyanion (i.e., a polymer that has a net negative charge at a specific pH). In a particular embodiment, the polyion segment has at least three charges, preferably at least 10 charges, and more preferably at least 15 charges. In a preferred embodiment, the charges are spaced close to each other. Indeed, without being bound by theory, it is believed that when the distance between polyelectrolyte charges is less than a certain critical value, the small counterions present in solution may condense onto a chain of such polyelectrolyte. For example, the "Bjerrum length" in aqueous solution of polyelectrolytes is about 7 angstrom (see Manning (1980) Biopolymers, 19:37-59). Such counterions may release into external solution during reaction of a polyelectrolyte with an oppositely charged polyion and thus may provide a "driving force" for formations of polyelectolyte complexes (Kabanov et al. (2002) Structure, dispersion stability and dynamics of DNA and polycation complexes. In Pharmaceutical Perspectives of Nucleic Acid-Based Therapeutics (S.W. Kim, R. Mahato, Eds.) Taylor & Francis, London, New York, pp. 164-189).

The degree of polymerization of the polyion segments is typically between about 10 and about 100,000. More preferably, the degree of polymerization is between about 20 and about 10,000, still more preferably between about 10 and about 1,000, and yet still more preferably between about 10 and about 200. Independently from the polyion segment, the degree of polymerization of the nonionic water soluble polymer segment is about 10 and about 100,000. More preferably, the degree of polymerization is between about 20 and about 10,000, still more preferably between about 10 and about 1,000, and yet still more preferably between about 10 and about 200.

The polyion segment encompasses polycation segments and polyanion segments. Examples of polycation segments include but are not limited to polymers and copolymers and their salts comprising units deriving from one or more monomers including, without limitation, primary, secondary and/or tertiary amines, each of which can be partially or completely quaternized, thereby forming quaternary ammonium salts. Examples of these monomers include cationic aminoacids (e.g., lysine, arginine, histidine, ornithine and the like), alkyleneimines (e.g., ethyleneimine, propyleneimine, butileneimine, pentyleneimine, hexyleneimine, spermine, and the like), vinyl monomers (e.g., vinylcaprolactam, vinylpyridine, and the like), acrylates and methacrylates (e.g., N,N-dimethylaminoethyl acrylate, N,N-dimethylaminoethyl methacrylate, N,N-diethylaminoethyl acrylate, N,N-diethylaminoethyl methacrylate, t-butylaminoethyl methacrylate, acryloxyethyltrimethyl ammonium halide, acryloxyethyl_{¬}dimethylbenzyl ammonium halide, methacrylamidopropyltrimethyl ammonium halide and the like), allyl monomers (e.g., dimethyl diallyl ammoniam chloride), aliphatic, heterocyclic or aromatic ionenes.

The polycations and polycation segments can be produced by polymerization of monomers that themselves may be not cationic, such as for example, 4-vinylpyridine, and then converted into a polycation form by various chemical reactions of the monomeric units, for example alkylation, resulting in appearance of ionizable groups. The conversion of the monomeric units can be incomplete resulting in a copolymer having a portion of the units that do not have ionizable groups, such as for example, a copolymer of vinylpyridine and N-alkylvinylpyridinuim halide.

Polycation segments can be a copolymer containing more than one type of monomeric units including a combination of cationic units with at least one other type of unit including, for example, cationic units, anionic units, zwitterionic units, hydrophilic nonionic units and/or hydrophobic units. Such polycation segments can be obtained by copolymerization of more than one type of chemically different monomers. When such a copolymer is employed, the charged groups should be spaced close enough together so that, when reacted with the other components, a complex is formed. In a preferred embodiment, the portion of non-cationic units is relatively low so that the polymer or polymer block remains largely cationic in nature. The polycation-containing polymer may be a blend of two or more polymers of different structures, such as polymers containing different degrees of polymerization, backbone structures, and/or functional groups.

Examples of polyanion segments include, but are not limited to, polymers and their salts comprising units deriving from one or more monomers including: unsaturated ethylenic monocarboxylic acids, unsaturated ethylenic dicarboxylic acids, ethylenic monomers comprising a sulfonic acid group, their alkali metal, and their ammonium salts. Examples of these monomers include acrylic acid, methacrylic acid, aspartic acid, alpha-acrylamidomethylpropanesulphonic acid, 2-acrylamido-2-methylpropanesulphonic acid, citrazinic acid, citraconic acid, trans-cinnamic acid, 4-hydroxy cinnamic acid, trans-glutaconic acid, glutamic acid, itaconic acid, fumaric acid, linoleic acid, linolenic acid, maleic acid, nucleic acids, trans-beta-hydromuconic acid, trans-trans-muconic acid, oleic acid, 1,4-phenylenediacrylic acid, phosphate 2-propene-1-sulfonic acid, ricinoleic acid, 4-styrene sulfonic acid, styrenesulphonic acid, 2-sulphoethyl methacrylate, trans-traumatic acid, vinylsulfonic acid, vinylbenzenesulphonic acid, vinyl phosphoric acid, vinylbenzoic acid and vinylglycolic acid and the like as well as carboxylated dextran, sulphonated dextran, heparin and the like. The examples of polyanions include, but are not limited to, polymaleic acid, polyamino acids (e.g., polyaspartic acid, polyglutamic acid, and their copolymers) polyacrylic acid, polymethacrylic acid, and the like.

The polyanions and polyanion segments can be produced by polymerization of monomers that themselves may not be anionic or hydrophilic, such as for example, tert-butyl methacrylate or citraconic anhydride, and then converted into a polyanion form by various chemical reactions of the monomeric units, for example hydrolysis, resulting in ionizable groups. The conversion of the monomeric units can be incomplete resulting in a copolymer having a portion of the units that do not have ionizable groups, such as for example, a copolymer of tert-butyl methacrylate and methacrylic acid.

The polyanion segment can be a copolymer containing more than one type of monomeric units including a combination of anionic units with at least one other type of units including anionic units, cationic units, zwitterionic units, hydrophilic nonionic units and/or hydrophobic units. Such polyanions and polyanion segments can be obtained by copolymerization of more than one type of chemically different monomers. When such a copolymer is employed, the charged groups should be spaced close enough together so that, when reacted with the other components, a complex is formed. In a preferred embodiment, the portion of non-anionic units is relatively low so that the polymer or polymer block remains largely anionic and hydrophilic in nature. The polyanion-containing polymer may be a blend of two or more polymers of different structures, such as polymers containing different degrees of polymerization, backbone structures, and/or functional groups.

In one preferred embodiment, the polyion segment is a polypeptide selected from the group consisting of polymers or copolymers of lysine, histidine, arginine, ornithine, aspartic acid and/or glutamic acid, and their salts. Examples of such synthetic polyions include polylysine, polyhistidine, polyarginine, polyornithine, polyaspartic acid, polyglutamic acid, and their salts. In another preferred embodiment, the polyion segment is selected from the group consisting of polyacrylic acid, polyalkylene acrylic acid, polyalkyleneimine, polyethylenimine, polyphosphates, and their salts.

The nonionic water soluble polymer segment may be selected from the group consisting of polyethylene oxide, a copolymer of ethylene oxide and propylene oxide, a polysaccharide, a polyacrylamide, a polygycerol, a polyvinylalcohol, a polyvinylpyrrolidone, a polyvinylpyridine N-oxide, a copolymer of vinylpyridine N-oxide and vinylpyridine, a polyoxazoline, and a polyacroylmorpholine, or derivatives thereof. Preferably, nonionic polymer segments are nontoxic and nonimmunogenic. In a particular embodiment, the water soluble polymers are poly(ethylene oxide) (PEO); poly(ethylene glycol) (PEG); or a copolymer of ethylene oxide and propylene oxide. If the nonionic water soluble polymer segment is poly(ethylene oxide), the preferred molecular mass of such polymer is between about 300 and about 20,000, more preferred between about 1,500 and about 15,000, still more preferred between about 2,000 and about 10,000, and yet still more preferred about 4,000 and about 10,000.

The polyion segment and nonionic water soluble polymer segment may contain different end groups. For example, the method of synthesis may lead to the inclusion of different end groups.

The complexes of the instant invention spontaneously self-assemble into particles of nanoscale size. Without being bound by theory, it is believed that the formed particles have a core-shell morphology. The core of the particles comprises the protein-polyion complex and the hydrophilic shell comprises the nonionic water soluble segment of the copolymer. Indeed, neutralization of the polyion charges leads to the formation of hydrophobic domains, which tend to segregate in aqueous media. However, the water-soluble nonionic segments prevent aggregation and macroscopic phase separation. As a result, these complexes self-assemble into particles of nanoscale size and form stable aqueous dispersions.

To build a protective nanocontainer for a polypeptide or protein of interest, block copolymers are synthesized by conjugation of a polyion segment (e.g., polyethylenimine (PEI, 2,000 Da)) and a nonionic water soluble segment (e.g., poly(ethylene oxide) (PEO, 10,000 Da) (Vinogradov et al. (1999) Bioconjug. Chem., 10:851-60). Complexes can be formed by the addition of a solution of the protein of interest (e.g., catalase (1 mg/ml)) to a solution of a block copolymer (e.g., PEI-PEG (2 mg/ml)) in a buffer (e.g., phosphate buffer saline (pH 7.4)) producing slightly opalescent dispersions.

The particles may be administered to a cell of the body in the isotonic solution at physiological pH 7.4. However, the complexes can be prepared before administration at pH below or above pH 7.4. It is recognized that many polypeptides of interest in this invention are polyampholytes, which contain both positive and negative groups. The balance of the positive and negative groups of such polypeptide depend on their chemical structure as well as on the pH of the external solution. At pH below the isoelectric point (pI) the polypeptides may be positively charged. At pH above the pI the polypeptides may be negatively charged. Therefore, the complexes of according to this invention may be produced by reacting polypeptides below the pH point with polyanion. These complexes may be also prepared by reacting polypeptides above the pI with polycations. Following preparation of the complexes the pH of the solution may be changed to the desired pH, for example, pH 7.4 for further administration. In some cases, the polypeptides may contain sites or domains with multiple positive or negative groups closely positioned to one another. Such polypeptides may form complexes with oppositely charged polyions (e.g., polycations in case of sites with multiple negative groups in polypeptide or polyanions in case of sites with multiple positive groups) both below and above the pH.

The core of the complexes may be cross-linked. The cross-links can chemically link the functional groups of the polypeptide, of polyions or both polypeptides and polyions including links between the polypeptides and polyions. The cross-linkers may be cleavable or degradable and may cleave in the body or within the cell. Various methods of cross-linking known in the art can be applied for cross-linking (G. Hermanson, Bioconjugate Techniques, Elsevier, 1996, 785 p.). Examples of cross-linkers include, without limitation, 1-ethyl-3-(3-dimethylaminopropyl)carbodiimide (DEC), glutaraldehyde (GA), formaldehyde, divinyl sulfone, a polyanhydride, a polyaldehyde, a polyhydric alcohol, a carbodiimide, epichlorohydrin, ethylene glycol diglycidylether, butanediol diglycidylether, polyglycerol polyglycidylether, polyethylene glycol, polypropylene glycol diglycidylether, a bis- or poly-epoxy cross-linker (e.g., 1,2,3,4-diepoxybutane or 1,2,7,8-diepoxyoctane), and those recited in G. Hermanson (Bioconjugate Techniques, Elsevier, 1996).

The cross linking ratio of the polypeptide-polyion complex may be from about 40% to about 75%, preferably about 40% to about 60%, and more preferably about 40% to about 50%. The presence of an excess of block copolymer in the polypeptide-polyion complexes can reduce the cross-linking ratio required for complex stability.

The polypeptide-polyion complexes of the instant invention may be administered to a mammalian subject, particularly a human. The polypeptide-polyion complexes of the instant invention are shown hereinbelow to be capable of crossing the BBB and delivering the polypeptide of interest to the CNS, particularly when the patient has a neurodegenerative or neuroinflammatory disease or disorder. Without being bound by theory, the polypeptide-polyion complex particles, following administration to the body of the mammalian subject, may be taken up into circulating cells capable of reaching the brain and a portion of the polypeptide is delivered to the brain by these cells. More specifically, the circulating cell may be an immune system cell such as a monocyte or a macrophage, preferably a bone marrow derived monocyte, a dendritic cell, a lymphocyte, preferably a T-cell, a neutrophil, an eosinophil a basophil, and combinations thereof.

Furthermore, without being bound by theory, it is believed that the complexes of the current invention provide protection to the polypeptide within the cells. At the same time, due to the specific core-shell structure induced by the block copolymer, the complexes are not toxic to the host cell and do not impair the functional properties of the cell. In particular, the complexes do not impair the ability of the cells to go to the site of the disease.

Without being bound to a theory, it is also believed that complexes may have increased circulation time alone or being entrapped in circulating cells. As a result, there may be an increased exposure of the circulating complexes to the BBB and increased percentage of the injected dose of the polypeptide delivered to the brain. Many disease conditions may result in decreased permeability of the BBB. This may further increase brain delivery of polypeptides.

Furthermore, without being bound to a theory, it is also believed that complexes may bind to and enter inside neuronal cells and/or neuronal peripheral projections and be transported to the brain through the process known as retrograde transport (Zweifel et al. (2005) Nat. Rev. Neurosci., 6:615-625; U.S. Patent Application Publication 2003/0083299) or a similar process. The unique structure of the complexes of the presence invention and, in particular, combination of ionic and non-ionic polymeric chains in the copolymers provides protection to the polypeptides, minimizes damage to cells and tissues, and facilitates free migration of the complexes to the brain.

The polypeptide-polyion complexes of the instant invention can be administered parenterally including, but not limited to, subcutaneously, intravenously and intraperitoneally. In addition, the polypeptide-polyion complexes may be administered directly to the nervous system, in particular intrathecally, intracerbrally or epidurally. The polypeptide-polyion complexes may also be administered intramuscularly, intradermally, or intracarotidly. A combination of different methods of administration may be used.

In accordance to another embodiment of the instant invention, the polypeptide-polyion complex is loaded into a cell, which can then be administered to a patient as a therapeutic agent. More specifically, the cell is a circulating cell, in particular, an immune system cell. Immune system cells include, without limitation, a monocyte, a macrophage, a bone marrow derived monocyte, a dendritic cell, a lymphocyte, a T-cell, a neutrophil, an eosinophil, a basophil, and/or combinations thereof. The loaded cells are capable of crossing the BBB and delivering the polypeptide of interest, particularly when the patient has a neurodegenerative or neroinflammatory disease or disorder. The cells may be isolated from the mammalian subject using cell isolation and separation techniques available in the art. As described hereinbelow, the cells can be loaded with the polypeptide-polyion complex by incubating the cell with the polypeptide-polyion complex. The loaded cells can be administered parenterally including, but not limited to, subcutaneously, intravenously and intraperitoneally. In addition to that they can be administered directly to the nervous system, in particularly intrathecally, intracerbrally or epidurally. The polypeptide-polyion complexes may also be administered intramuscularly, intradermally, or intracarotidly. A combination of different methods of administration may be used.

Neuroinflammation, perpetrated through activation of brain mononuclear phagocytes (MP; perivascular and parenchymal macrophages and microglia) along with astrocytes and endothelial cells, may act through paracrine pathways to accelerate neuronal injury in highly divergent diseases such as Alzheimer's disease (AD) and Parkinson's disease (PD), Huntington's diseases (HD), HIV associated neurocognitive disorders (HAND), and spongiform encephalopathies and stroke. In these disorders, CNS inflammatory infiltrates are complex and multifaceted. The initial responders or the MP cell elements of innate immunity set up a cascade, which later involves the activation and recruitment of the adaptive immune system and ultimately neurodegeneration. On balance, microglia are the primary MPs in the CNS that respond to injury and whose principal function is brain defense. Activated microglia participate in inflammatory processes linked to neurodegeneration by producing neurotoxic factors including quinolinic acid, superoxide anions, matrix metalloproteinases (MMP), nitric oxide, arachidonic acid and its metabolites, chemokines, pro-inflammatory cytokines and excitotoxins including glutamate. On the other hand, neuroprotective functions of microglia may be mediated through their abilities to produce neurotrophins and to scavange and eliminate excitotoxins present in the extracellular spaces. Indeed, neuronal survival after brain injury is known to be positively affected by microglial activities. Without limiting the instant invention to a specific theory, it is believed that these common mechanisms for neurodegeneration can be used for therapeutic gain using immune cells carriage of polypeptide-polyion complexes. In a preferred method
mononuclear phagocytes are used that have an extraordinary ability to cross the BBB due to their margination and extravasation properties.

An exemplary method
comprises: isolating target cell from a patient, incubating the isolated cells with polypeptide-polyion complexes, and injecting the cells back into the patient. Without limiting the instant invention to a specific theory, it is believed that one factor for this approach is the ability of polypeptide-polyion complexes to protect its load against proteolysis, which is extremely aggressive in phagocytes' lysosomes. It is further believed that core-shell polypeptide-polyion complexes do not change the ability of circulating cells to cross the BBB and carry the payload to the brain.

### I. Definitions

The following definitions are provided to facilitate an understanding of the present invention:
As used herein, the term "polymer" denotes molecules formed from the chemical union of two or more repeating units or monomers. The term "block copolymer" most simply refers to conjugates of at least two different polymer segments, wherein each polymer segment comprises two or more adjacent units of the same kind.

The term "isolated protein" or "isolated and purified protein" is sometimes used herein. This term refers primarily to a protein produced by expression of an isolated nucleic acid molecule of the invention. Alternatively, this term may refer to a protein that has been sufficiently separated from other proteins with which it would naturally be associated, so as to exist in "substantially pure" form. "Isolated" is not meant to exclude artificial or synthetic mixtures with other compounds or materials, or the presence of impurities that do not interfere with the fundamental activity, and that may be present, for example, due to incomplete purification, or the addition of stabilizers.

"Polypeptide" and "protein" are sometimes used interchangeably herein and indicate a molecular chain of amino acids. The term polypeptide encompasses peptides, oligopeptides, and proteins. The terms also include post-expression modifications of the polypeptide, for example, glycosylations, acetylations, phosphorylations and the like. In addition, protein fragments, analogs, mutated or variant proteins, fusion proteins and the like are included within the meaning of polypeptide.

The term "isolated" may refer to protein, nucleic acid, compound, or cell that has been sufficiently separated from the environment with which it would naturally be associated, so as to exist in "substantially pure" form. "Isolated" does not necessarily mean the exclusion of artificial or synthetic mixtures with other compounds or materials, or the presence of impurities that do not interfere with the fundamental activity, and that may be present, for example, due to incomplete purification.

"Pharmaceutically acceptable" indicates approval by a regulatory agency of the Federal or a state government or listed in the U.S. Pharmacopeia or other generally recognized pharmacopeia for use in animals, and more particularly in humans.

A "carrier" refers to, for example, a diluent, adjuvant, preservative (e.g., Thimersol, benzyl alcohol), anti-oxidant (e.g., ascorbic acid, sodium metabisulfite), solubilizer (e.g., Tween 80, Polysorbate 80), emulsifier, buffer (e.g., Tris HCl, acetate, phosphate), water, aqueous solutions, oils, bulking substance (e.g., lactose, mannitol), excipient, auxilliary agent or vehicle with which an active agent of the present invention is administered. Suitable pharmaceutical carriers are described in "Remington's Pharmaceutical Sciences" by E.W. Martin (Mack Publishing Co., Easton, PA); Gennaro, A. R., Remington: The Science and Practice of Pharmacy, 20th Edition, (Lippincott, Williams and Wilkins), 2000; Liberman, et al., Eds., Pharmaceutical Dosage Forms, Marcel Decker, New York, N.Y., 1980; and Kibbe, et al., Eds., Handbook of Pharmaceutical Excipients (3rd Ed.), American Pharmaceutical Association, Washington, 1999.

### II. Therapeutic agent

While the the instant invention involves proteins contained within the polymer complex, it is also possible
to encapsulate other therapeutic agents or compounds of interest into the polymer complex. Such agents or compounds include, without limitation, polypeptides, peptides, nucleic acids, and compounds such as synthetic and natural drugs. In a preferred embodiment, the therapeutic agent is a polypeptide or protein. While the description of the instant invention references polypeptide-polyion complexes throughout, the use of proteins is also contemplated within the instant invention. In many cases, the terms polypeptide and protein are used herein interchangeably.

In a preferred embodiment of the instant invention, the protein of interest in the polymer complex is a therapeutic protein, i.e., it effect amelioration and/or cure of a disease, disorder, pathology, and/or the symptoms associated therewith. The proteins may have therapeutic value against neurological disorders (particularly of the CNS) including, without limitation, neurological degenerative disorders, Alzheimer's disease, Parkinson's disease, Huntington's disease (HD), stroke, trauma, infections, meningitis, encephalitis, gliomas, cancers (including brain metastasis), HIV-1 associated dementia (HAD), HIV associated neurocognitive disorders (HAND), paralysis, amyotrophic lateral sclerosis (ALS or Lou Gerhig's disease), multiple sclerosis (MS), CNS-associated cardiovascular disease, prion disease, obesity, metabolic disorders, inflammatory disease, metabolic disorders, and lysosomal storage diseases (LSDs; such as, without limitation, Gaucher's disease, Pompe disease, Niemann-Pick, Hunter syndrome (MPS II), Mucopolysaccharidosis I (MPS I), GM2-gangliosidoses, Gaucher disease, Sanfilippo syndrome (MPS IIIA), Tay-Sachs disease, Sandhoff's disease, Krabbe's disease, metachromatic leukodystrophy, and Fabry disease). Therapeutically active proteins include but are not limited to enzymes, antibodies, hormones, growth factors, other polypeptides, which administration to the brain can effect amelioration and/or cure of a disease, disorder, pathology, and/or the symptoms associated therewith. Neuroactive polypeptides useful in this invention include but are not limited to endocrine factors, growth factors, hypothalamic releasing factors, neurotrophic factors, paracrine factors, neurotransmitter polypeptides, antibodies and antibody fragments which bind to any of the above polypeptides (such neurotrophic factors, growth factors, and others), antibodies and antibody fragments which bind to the resecptors of these polypeptides (such as neurotrophic factor receptors), cytokines, endorphins, polypeptide antagonists, agonists for a receptor expressed by a CNS cell, polypeptides involved in lysosomal storage diseases, and the like. In a particular embodiment, the therapeutic protein exerts its effect on the CNS. In another particular embodiment, the therapeutic protein does not cross the BBB by itself.

Examples of specific proteins include, without limitation, catalase, telomerase, superoxidedismutase (SOD), glutathionperoxidase, glutaminase, cytokines, endorphins (e.g. enkephalin), growth factors (e.g., epidermal growth factor (EGF), acidic and basic fibroblast growth factor (aFGF and bFGF), insulin-like growth factor I (IGF-I), brain-derived neutrotrophic factor (BDNF), glial-derived neutrotrophic factor (GDNF), platelet derived growth factor (PDGF), vascular growth factor (VGF), nerve growth factor (NGF), insulin-like growth factor-II (IGF-II), tumor necrosis factor-B (TGF-B), leukemia inhibitory factor (LIF), various interleukins, and the like), antiapoptotic proteins (BCL-2, PI3 kinase, and the like), amyloid beta binders (e.g. antibodies), modulators of α-, β-, and/or γ-secretases, vasoactive intestinal peptide, leptin, acid alpha-glucosidase (GAA), acid sphingomyelinase, iduronate-2-sultatase (I2S), α-L-iduronidase (IDU), β-Hexosaminidase A (HexA), Acid β-glucocerebrosidase, N-acetylgalactosamine-4-sulfatase, α-galactosidase A, and neurotransmitters (see, e.g., Schapira, A.H. (2003) Neurology 61:S56-63; Ferrari et al. (1990) Adv Exp Med Biol. 265:93-99; Ferrari et al. (1991) J Neurosci Res. 30:493-497; Koliatsos et al. (1991) Ann Neurol. 30:831-840; Dogrukol-Ak et al. (2003) Peptides 24:437-444; Amalfitano et al. (2001) Genet Med. 3:132-138; Simonaro et al. (2002) Am J Hum Genet. 71:1413-1419; Muenzer et al. (2002) Acta Paediatr Suppl. 91:98-99; Wraith et al. (2004) J Pediatr. 144:581-588; Wicklow et al. (2004) Am J Med Genet. 127A:158-166; Grabowski (2004) J Pediatr. 144:S15-19; Auclair et al. (2003) Mol Genet Metab. 78:163-174; Przybylska et al. (2004) J Gene Med. 6:85-92). Lysosomal storage diseases are inherited genetic defects that result in an enzyme deficiency, which prevents cells from performing their natural recycling function (Enns and Huhn, (2008) Neurosurg. Focus 24:E12). This leads to a variety of progressive physical and/or mental deterioration and it is believed that delivery of these deficient enzymes to the brain can result in treatment of these diseases. Various enzymes implicated in lysosomal storage diseases or enzymes that can fulfill the function of the deficient enzymes can be delivered using the methods described herein.

The polypeptide complexes described herein can be used as a treatment modality against acute nerve toxicity from warfare agents based on the brain delivery of butyrylcholinesterase or acetylcholinesterase, cholinesterase reactivators (e.g., oxime compounds), scavengers of organophosphate and carbamate inhibitors. Since butyrylcholinesterase (BChE) also hydrolyzes many ester-containing drugs, such as cocaine and succinylcholine, the BChE within complexes of this invention has therapeutic value against cocaine addiction and toxicity (e.g., Carmona et.al. (1999) Drug Metab. Dispos., 28:367-371; Carmona (2005) Eur. J. Pharmacol., 517:186-190).

The methods described herein involve the use of polypeptide complexes containing one or several useful polypeptides, or use of several complexes containing different polypeptides that can be administered alone or with cells, simultaneously or separately from each other. The complexes may be in the same composition or may be in separate compositions.

### III. Administration

The polypeptide-polyion complexes and the cells comprising the polypeptide-polyion complex described herein will generally be administered to a patient as a pharmaceutical preparation. The term "patient" as used herein refers to human or animal subjects. These polypeptide-polyion complexes and the cells comprising the same may be employed therapeutically, under the guidance of a physician.

The pharmaceutical preparation comprising the polypeptide-polyion complexes and/or cells loaded with the polypeptide-polyion complex of the invention may be conveniently formulated for administration with any pharmaceutically acceptable carrier. For example, the complexes and cells may be formulated with an acceptable medium such as water, buffered saline, ethanol, polyol (for example, glycerol, propylene glycol, liquid polyethylene glycol and the like), dimethyl sulfoxide (DMSO), oils, detergents, suspending agents or suitable mixtures thereof. The concentration of the polypeptide-polyion complexes and/or the cells in the chosen medium may be varied and the medium may be chosen based on the desired route of administration of the pharmaceutical preparation. Except insofar as any conventional media or agent is incompatible with the polypeptide-polyion complexes or cells to be administered, its use in the pharmaceutical preparation is contemplated.

The dose and dosage regimen of polypeptide-polyion complexes and/or cells according to the invention that are suitable for administration to a particular patient may be determined by a physician considering the patient's age, sex, weight, general medical condition, and the specific condition for which the polypeptide-polyion complex or cell is being administered and the severity thereof. The physician may also take into account the route of administration, the pharmaceutical carrier, and the polypeptide-polyion complex's or cell's biological activity.

Selection of a suitable pharmaceutical preparation will also depend upon the mode of administration chosen. For example, the polypeptide-polyion complex or cell comprising the polypeptide-polyion complex of the invention may be administered by direct injection into an area proximal to the blood brain barrier. In this instance, a pharmaceutical preparation comprises the polypeptide-polyion complex or cells dispersed in a medium that is compatible with the site of injection.

Polypeptide-polyion complexes or cells of the instant invention may be administered by any method such as intravenous injection into the blood stream, oral administration, or by subcutaneous, intramuscular or intraperitoneal injection. Pharmaceutical preparations for injection are known in the art. If injection is selected as a method for administering the polypeptide-polyion complex or cells, steps must be taken to ensure that sufficient amounts of the molecules or cells reach their target cells to exert a biological effect.

Pharmaceutical compositions containing a complex or cell of the present invention as the active ingredient in intimate admixture with a pharmaceutically acceptable carrier can be prepared according to conventional pharmaceutical compounding techniques. The carrier may take a wide variety of forms depending on the form of preparation desired for administration, e.g., intravenous, oral, direct injection, intracranial, and intravitreal.

A pharmaceutical preparation of the invention may be formulated in dosage unit form for ease of administration and uniformity of dosage. Dosage unit form, as used herein, refers to a physically discrete unit of the pharmaceutical preparation appropriate for the patient undergoing treatment. Each dosage should contain a quantity of active ingredient calculated to produce the desired effect in association with the selected pharmaceutical carrier. Procedures for determining the appropriate dosage unit are well known to those skilled in the art.

Dosage units may be proportionately increased or decreased based on the weight of the patient. Appropriate concentrations for alleviation of a particular pathological condition may be determined by dosage concentration curve calculations, as known in the art.

The appropriate dosage unit for the administration of polypeptide-polyion complexes or cells containing the complexes may be determined by evaluating the toxicity of the molecules or cells in animal models. Various concentrations of polypeptide-polyion complexes or cells in pharmaceutical preparations may be administered to mice, and the minimal and maximal dosages may be determined based on the beneficial results and side effects observed as a result of the treatment. Appropriate dosage unit may also be determined by assessing the efficacy of the polypeptide-polyion complex or cell treatment in combination with other standard drugs. The dosage units of polypeptide-polyion complex may be determined individually or in combination with each treatment according to the effect detected.

The pharmaceutical preparation comprising the polypeptide-polyion complexes or cells may be administered at appropriate intervals, for example, at least twice a day or more until the pathological symptoms are reduced or alleviated, after which the dosage may be reduced to a maintenance level. The appropriate interval in a particular case would normally depend on the condition of the patient.

The following examples provide illustrative methods of practicing the instant invention, and are not intended to limit the scope of the invention in any way.

### EXAMPLE 1:

The need for delivery of therapeutic polypeptides to affected brain tissues in Alzheimer's and Parkinson's diseases (AD and PD) (Brinton, R.D. (1999) Int. J. Fertil. Womens Med., 44:174-85; Gozes, I. (2001) Trends Neurosci., 24:700-5; Kroll et al. (1998) Neurosurgery 42:1083-100), infections (meningitis, encephalitis, prion disease, and HIV-related dementia) (Bachis et al. (2005) Ann. N. Y. Acad. Sci., 1053:247-57; Wang et al. (2003) Virology 305:66-76), stroke (Koliatsos et al. (1991) Ann. Neurol., 30:831-40; Dogrukol-Ak et al. (2003) Peptides 24:437-44), lysosomal storage (Desnick et al. (2002) Nat. Rev. Genet., 3:954-66; Urayama et al. (2004) Proc. Natl. Acad. Sci., 101:12658-63), obesity (Banks, W. (2003) Curr. Pharm. Des., 9:801-809; Banks et al. (2002) J. Drug Target., 10:297-308), and other metabolic and inflammatory diseases of the CNS is immediate and cannot be overstated.

An important component of metabolic and degenerative diseases of the nervous system involves inflammation (Perry et al. (1995) Curr. Opin. Neurobiol., 5:636-41). Such inflammatory activities are profound, as they lead to excessive production of pro-inflammatory products and reactive oxygen species (ROS) that lead in part, to cell death and neurodegeneration. By affecting neuroinflammatory activities during disease, such as through the use of targeted antioxidants or drugs that inhibit the production or formation of proinflammatory cytokines and eicosanoids, the levels of ROS as well as other neurotoxins can be reduced, resulting in improved disease outcomes (Prasad, et al. (1999) Curr. Opin. Neurol., 12:761-70). However, such approaches have been limited, as drugs must not only penetrate the BBB but also find themselves in sufficient concentrations to affect ongoing disease mechanisms. Moreover, as inflammatory mechanisms are a likely early event for disease, therapeutic modalities must be used early and frequently. The limitation of drug delivery is one major obstacle confronting the development of new treatment paradigms for nervous system disorders.

One such disease is PD, the second most prevalent neurodegenerative disorder in people over 65. This disease is characterized by lack of the neurotransmitter dopamine due to a loss of dopaminergic neurons within the SNpc and their innervations to the striatum. PD neuropathology involves brain inflammation, microglia activation, and subsequent secretory neurotoxic activities, including ROS production, that play crucial roles in cell damage and death (McGeer et al. (1988) Neurology 38:1285-91; Busciglio et al. (1995) Nature 378:776-9; Ebadi et al. (1996) Prog. Neurobiol., 48:1-19; Wu et al. (2003) Proc. Natl. Acad. Sci., 100:6145-50). PD brains show reduced levels of antioxidant enzymes and antioxidants (Ambani et al. (1975) Arch. Neurol., 32:114-8; Riederer et al. (1989) J. Neurochem., 52:515-20; Abraham et al. (2005) Indian J. Med. Res., 121:111-5) resulting in a reduced capacity to manage oxidative stress and associated neurodegeneration. Mounting evidence supports the notion that antioxidants can inhibit inflammatory responses and protect dopaminergic neurons in laboratory and animal models of PD (Wu et al. (2002) J. Neurosci., 22:1763-71; Du et al. (2001) Proc. Natl. Acad. Sci., 98:14669-74; Kurkowska-Jastrzebska et al. (2002) Int. Immunopharmacol., 2:1213-8; Teismann et al. (2001) Synapse 39:167-74; Ferger et al. (1999) Naunyn Schmiedebergs Arch. Pharmacol., 360:256-61; Ferger et al. (1998) Naunyn Schmiedebergs Arch. Pharmacol., 358:351-9; Peng et al. (2005) J. Biol. Chem., 280:29194-8). Catalase catalyzes the conversion of hydrogen peroxide, a known ROS, to water and molecular oxygen with one of the highest turnover rates for all known enzymes. Mounting evidence suggests that antioxidants can inhibit the inflammatory response and protect up to 90% of dopaminergic neurons *in vitro* and *in vivo* (Wu et al. (2002) J. Neurosci., 22:1763-71; Du et al. (2001) Proc. Natl. Acad. Sci., 98:14669-74; Kurkowska-Jastrzebska et al. (2002) Int. Immunopharmacol., 2:1213-8; Teismann et al. (2001) Synapse 39:167-74; Ferger et al. (1999) Naunyn. Schmiedebergs Arch. Pharmacol., 360:256-61; Ferger et al. (1998) Naunyn. Schmiedebergs Arch. Pharmacol., 358:351-9; Peng et al. (2005) J. Biol. Chem., 280:29194-8). In an *in vitro* model of PD, catalase was shown to rescue primary cultured cerebellar granule cells from ROS toxic effects (Prasad et al. (1999) Curr. Opin. Neurol., 12:761-70; Gonzalez-Polo et al. (2004) Cell Biol. Int., 28:373-80). Furthermore, a low molecular mass catalase activator, rasagiline, induced neuroprotection in a mouse model of PD (Maruyama et al. (2002) Neurotoxicol. Teratol., 24:675-82). Few clinical trials have been performed using low molecular mass antioxidants, of which the most extensive used is R-tocopherol and deprenyl to inhibit the rate of PD progression (Group, T.P.S. (1993) N. Engl. J., 328:176-183). However, and as described above, most of the trials failed to show significant improvements because of restricted transport of R-tocopherol across the BBB and the time following the disease the drugs were used (Pappert et al. (1996) Neurology, 47:1037-42).

### Materials and Methods

*Materials.* Catalase from bovine liver, polyethylenimine (PEI) (2K, branched, 50% aq solution), sulforhodamine-B (SRB), sodium dodecylsulfate (SDS), Sephadex G-25, and Triton X-100 were purchased from Sigma-Aldrich (St-Louis, MO). Methoxypoly(ethylene glycol) epoxy (Me-PEG-epoxy) was purchased from Shearwater Polymer Inc., Huntsville, AL.

*MPTP.* For 1-methyl-4-phenyl-1,2,3,6-tetrahydropyridine (MPTP)-intoxication recipient C57BL/6, mice were treated as described (Benner et al. (2004) Proc. Natl. Acad. Sci., 101:9435-40). After 12 hours, MPTP-treated mice were injected i.v. with the 50 µCi/mouse of ¹²⁵I-labeled polypeptide-polyion complex. After 24 hours mice were sacrificed and the amount of radioactivity in major organs (brain, spleen, liver, lungs, and kidney) was detected by 1480 gamma-counter Wizard 3 (Perkin-Elmer Life Sciences, Shelton, CT). The amount of the delivered enzyme was expressed as a percent of the injected dose for the whole organ.

*PEI-PEG Conjugates.* The copolymer was synthesized using a modified procedure (Nguyen et al. (2000) Gene Ther., 7:126-38) by conjugation of PEI and Me-PEG-epoxy. Briefly, Me-PEG-epoxy water solution was added to 5% PEI in water and incubated overnight at room temperature. To purify from the excess of PEI (as well as from low molecular weight residuals), the obtained conjugates were dialyzed in SpectraPore membrane tubes with cutoff 6000-8000 Da against water (twice replaced) for 48 hours and then concentrated *in vacuo.* For final purification, the conjugate was dissolved in 20 mL of 100% methanol and then added dropwise to 400 mL of ether. The precipitate was centrifuged (400g, 5 minutes), washed twice with ether, and dried in an exicator. Detailed characterization of the product was performed by spectrophotometry and mass spectrometry as reported (Nguyen et al. (2000) Gene Ther., 7:126-38).

*Block Ionomer Complexes.* Given amounts of the catalase (1 mg/mL) and the block copolymer (2 mg/mL) were separately dissolved in phosphate-buffered saline (PBS) at room temperature. A solution of the enzyme was added dropwise to the block copolymer solution at constant stirring. The +/- charge ratio (Z) was calculated by dividing the amount of amino groups of PEI-PEG protonated at pH 7.4 (Vinogradov et al. (1998) Bioconjugate Chem., 9:805-812) by the total amount of Gln and Asp in catalase. A combination of physicochemical methods (electrophoretic retention, dynamic light scattering (DLS), and transmission electron microscopy (TEM)) was used to characterize composition, size, dispersion stability, morphology, shape, and structure of the obtained nanoparticles, as described previously (Vinogradov et al. (1999) Bioconjugate Chem., 10:851-60; Lemieux et al. (2000) J. Drug Target., 8:91-105; Vinogradov et al. (2004) J. Drug Target., 12:517-26; Vinogradov et al. (2005) J. Controlled Release, 107:143-57).

*Electrophoretic* Retention. The formation of polyion complexes was examined by acrylamide gel shift assay. Enzyme complexes at various Z were loaded in a 7.5% acrylamide gel with 5 mM Tris, 50 mM glycine, pH 8.3, under nondenaturizing conditions (in the absence of SDS) to preserve the complex. The protein bands were visualized with rabbit polyclonal anticatalase (Ab 1877, Abcam Inc, Cambridge, MA; 1:6000) and secondary horseradish peroxidase anti-rabbit Ig Ab (Amersham Life Sciences, Cleveland, OH; 1:1500). The specific protein bands were visualized using a chemiluminescence kit (Pierce, Rockford, IL).

*Light Scattering Measurements.* Effective hydrodynamic diameter and zeta-potential of polypeptide-polyion complexes was measured by photon correlation spectroscopy using 'ZetaPlus' Zeta Potential Analyzer (Brookhaven Instruments, Santa Barbara, CA) as described previously (Bronich et al. (2000) J. Am. Chem. Soc., 122:8339-8343; Vinogradov et al. (1999) Colloids Surf. B-Biointerfaces 16:291-304).

*TEM.* A drop of catalase/PEI-PEG dispersion (Z = 1) in PBS was placed on Formvar-coated copper grid (150 mesh, Ted Pella Inc., Redding, CA). The dried grid containing polypeptide-polyion complexes was stained with vanadyl sulfate and visualized using a Philips 201 transmission electron microscope (Philips/FEI Inc., Briarcliff
Manor, NY).

*Catalase and Catalase Activity.* The activity of the enzyme in polymer nanoparticles was studied using the reaction rate of hydrogen peroxide decomposition by catalase or catalase-polyion complexes at various charge ratios and was determined by monitoring the change in absorbance at 240 nm (the extinction coefficient of H₂O₂ is 44 X 10⁶ M⁻¹ cm¹).

*¹²⁵I-Labeling of Catalase-polyion complex.* To obtain ¹²⁵I-labeled catalase-polyion complex, the protein solution in PBS (1 mg/mL) was incubated for 15 minutes with Na¹²⁵I (1 mCi) in the presence of IODO-BEADS Iodination Reagent (Pierce, Rockford, IL) and then purified from nonconjugated label using D-salt Desalting Columns (Pierce, Rockford, IL). ¹²⁵I-labeled catalase (400 µCi/mL, 0.7 mg/mL) was supplemented with PEI-PEG block copolymer (Z = 1).

*Statistical Analysis.* For the all experiments, data are presented as the mean ± SEM. Tests for significant differences between the groups were done using one-way ANOVA with multiple comparisons (Fisher's pairwise comparisons) using GraphPad Prism 4.0 (GraphPad software, San Diego, CA). A minimum p value of 0.05 was estimated as the significance level for all tests.

### Results

Block ionomer complexes spontaneously form by mixing block ionomers with either oppositely charged surfactants or polyelectrolytes (Harada et al. (2001) J. Controlled Release 72:85-91; Kabanov et al. (1995) Bioconjugate Chem., 6:639-643; Harada et al. (1995) Macromolecules 28:5294-5299; Bronich et al. (1997) Macromolecules 30:3519-3525). Neutralization of the polyion charges leads to formation of hydrophobic domains, which segregate in aqueous media into a core of polyion complex micelles. Water-soluble nonionic segments of block ionomers (for example, PEG) prevent aggregation and macroscopic phase separation. As a result, these complexes self-assemble into particles of nanoscale size and form stable aqueous dispersions (Figure 1A). Catalase has a net negative charge under physiological conditions. Therefore, the polyion complexes were obtained in phosphate buffer (pH 7.4) by mixing the enzyme (1 mg/mL) and PEI-PEG (2 mg/ mL), which is positively charged.

Catalase and PEI-PEG complexes were obtained at various +/- charge ratios (Z = from 0 to 4). They were subjected to electrophoresis under nondenaturizing conditions and then transferred to nitrocellulose membranes. The protein bands were visualized with antibodies to catalase (Figure 1B). The band intensity decreased as the copolymer increased. This suggested that complexes formed that were unable to enter the gel and was confirmed by DLS. Addition of PEI-PEG to catalase solution (1 mg/mL) resulted in particles of nanoscale size with relatively low polydispersity index (about 0.1-0.2), while no particles were detected for catalase alone.

Particle size depended on the charge ratio, ionic strength, and pH (Figure 1, parts C, D, and E). In PBS, the effective diameter increased as the charge ratio increased and then stabilized at ca. 90 to 100 nm at the charge ratio (Z) of 1 and above (Figure 1C). The zeta-potential was increased upon increasing the amount of the block copolymer (Figure 1C). At a constant charge ratio (Z = 1) large aggregates over 600 nm were formed in the absence of salt (Figure 1D). Addition of salt decreased the particle size which stabilized at ca. 90 nm as the NaCl concentration reached 0.15 M. It is likely that large nonequilibrium polyelectrolyte complex aggregates form upon mixing the catalase and PEI-PEG solutions. In the absence of salt these aggregates could not equilibrate and remained "frozen" due to a low rate of polyion interchange (Kabanov, V. (1994) Polym. Sci., 36:143-156; Kabanov, V. (2003) Fundamentals of Polyelectrolyte Complexes in Solution and the Bulk. In Multilayer Thin Films (Decher, G., and Schlenoff, J., Eds.) pp 47-86, Wiley-VCH Verlag GmbH & Co. KGaA, Weinheim). As salt was added the polyion interchange was accelerated, resulting in formation of small (equilibrium) particles. These particles were stable in an approximate range of pH 7.4 to 11.5 but irreversibly aggregated when pH was decreased below or increased above this range (Figure 1E). Within this range the catalase and PEI-PEG were oppositely charged. The aggregation of the complexes was linked to protonation and charge inversion of catalase (pI = 6.5) at low or deprotonation of PEI at high pH. Overall, polypeptide-polyion complex particles were stable under physiological pH and ionic strength. Under these conditions the particles were close to spherical (Figure 1F). No changes in the enzymatic activity of catalase were observed at charge ratios used for subsequent cell loading, delivery, and release experiments (Figure 1G).

To determine if polypeptide-polyion complexes could reach brain subregions with active neuroinflammatory disease reflective of human PD, the MPTP model was used. MPTP causes a severe and irreversible Parkinsonian syndrome in humans and in nonhuman primates (Langston et al. (1986) Clin. Neuropharmacol. 9:485-507), initiating a self-perpetuating process of nigrostriatal neurodegeneration (Langston et al. (1999) Ann. Neurol. 46:598-605). In mice, MPTP reproduces most of the biochemical and pathological hallmarks of PD, including specific degeneration of dopaminergic neurons in the SNpc and corresponding striatum (Schmidt et al. (2001) J. Neural. Transm. 108:1263-82) and glial inflammation (Gao et al. (2003) Trends Pharmacol. Sci., 24:395-401).

MPTP-intoxicated C57Bl/6 mice were injected intravenously with free polypeptide-polyion complexes containing ¹²⁵I-labeled catalase. Twenty four hours after injection radioactivity was detectable in the brain, as well as other tissues.

### EXAMPLE 2

Cationic block ionomer of graft architecture, poly-L-lysine-graft-poly(ethylene oxide), PLL-g-PEO(2), containing ca. 1.4 PEO chains grafted onto a PLL backbone, was used to prepare butyrylcholine esterase BChE/PLL-g-PEO complexes. An estimated molecular mass of PLL-g-PEO(2) is ca. 24,000 g/mol according to ¹H NMR analysis. Both samples of human BChE (Hu BChE) and BChE from equine serum (Hor BChE) were used in this study.

Complexes of Hu BChE with PLL-g-PEO(2) were prepared by simple mixing of buffered solutions (phosphate buffer, 10 mM, pH 7.4) of the block ionomer and protein components. The compositions of mixtures close to stoichiometric charge ratio between the components were studied and presented in Table 1. Compositions of mixture were expressed in terms of SA/Lys molar ratio (calculated by dividing the concentration of amino groups of PLL-g-PEO(2) by the concentration of sialic acid units in BChE). The composition of the BChE/PLL-g-PEO(2) mixtures was also expressed in terms of total amount of carboxylic groups (Glu, Asp, and sialic acid) in protein and calculated as a ratio of concentration of amino group in PLL-g-PEO(2) to the total concentration of carboxylic groups in protein (Z+/-).

**Table 1**

| Sample | Hu BChE/PLL-g-PEO(2) (SA/Lys molar ratio) | Z+/- |
|---|---|---|
| 1 | 1:1 | 0.24 |
| 2 | 1:3 | 0.7 |
| 3 | 1:5 | 1.2 |

The extent of incorporation of Hu BChE into block ionomer complexes was monitored using non-denaturating polyacrylamide gel electrophoresis (PAGE). Figure 2A presents the gel electrophoresis pattern observed for Hu BChE and PLL-g-PEO(2) mixtures. The Hu BChE bands intensity was significantly decreased as the amount of the copolymer in the mixture was increased. This demonstrated that the PLL-g-PEO(2) copolymer was binding to the Hu BChE, neutralizing its charge. Practically complete retardation of complex migration was observed at the composition of Hu BChE/PLL-g-PEO(2) mixtures in the vicinity of Z+/- = 1.0.

Complexes of Hor BChE and PLL-g-PEO(2) were prepared in a similar way and compositions of the mixtures are presented in Table 2. The extent of incorporation of Hor BChE into block ionomer complexes was monitored using non-denaturating PAGE. Figure 2B presents the gel electrophoresis pattern observed for Hor BChE and PLL-g-PEO(2) mixtures. The complete immobilization of Hor BChE into the complexes was observed at the excess of block ionomer in the mixtures (Z+/-=6.2). Similar data was obtained for the complexes of Hor BChE and PLL-g-PEO copolymer with grafting density of PEO ca. 6.6 chains per PLL chain (designated as PLL-g-PEO(7)).

**Table 2**

| Sample | Hor BChE/PLL-g-PEO(2) (SA/Lys molar ratio) | Z+/- |
|---|---|---|
| 1 | 1:5 | 1.2 |
| 2 | 1:10 | 2.3 |
| 3 | 1:15 | 3.4 |
| 4 | 1:27 | 6.2 |
| 5 | 1:36 | 8.2 |
| 6 | 1:45 | 10.3 |

The complexes of BChE of both types and PLL-g-PEO(2) were further characterized by dynamic light scattering. The data for all types of complexes studies are summarized in Figure 3. Particles of slightly larger size than protein alone were detected in all BChE/block ionomer mixtures.

Molecular mass (Mw) for Hu BChE/PLL-g-PEO(2) complexes was measured via sedimentation equilibrium analysis. All measurements were made at 20°C at rotor speed of 4000 rpm during sedimentation time of 24 hour. Resulting sedimentation equilibrium pattern were recorded with an UV absorbance optical system. An average protein partial specific volume of 0.73 cm³/g was used for calculation of molecular weights from measured sedimentation equilibria. The calculated molecular masses are presented in Table 3.

**Table 3**

| Sample | Z_{+/-} | M_{w} | Variance |
|---|---|---|---|
| Hu BChE | - | 364,215 | 1.14 x 10⁻⁵ |
| Hu BChE/PLL-g-PEO(2) | 1.2 | 420,489 | 1.18 x 10⁻⁵ |
| Hu BChE/PLL-g-PEO(2) | 6.2 | 450,509 | 1.0 x 10⁻⁵ |

These data also suggest that complexes formed from Hu BChE and PLL-g-PEO(2) consist of one molecule of protein. The observed increase in molecular mass of the complexes compare to protein alone corresponds to the binding of ca. 2-3 chains of PLL-g-PEO(2) copolymer per protein tetramer.

The activity of Hu BChE incorporated into the complexes was determined using assay based on the hydrolysis of butyrylthiocholine iodide and is presented in Table 4. No changes in enzymatic activity of BChE incorporated in the complexes were observed even in presence of the excess of block ionomer. Since very low concentrations of enzyme or complex (0.0025 mg/ml on BChE base) are required for determination of BChE activity, it was necessary to confirm that complexes remain their integrity at such dilutions. The complexes at various dilutions were examined using PAGE technique followed by Karnovsky & Roots activity stain of the gel (Karnovsky and L. Roots (1964) J. Histochem, Cytochem, 12:219-221). This "direct-coloring" thiocholine method is highly sensitive at low concentration of BChE. A typical gel electrophoresis pattern is presented in Figure 4A. These data indicate that complexes of BChE and block ionomer dissociate when greatly diluted.

**Table 4**

| Z_{+/-} | Activity(units/mg) | |
|---|---|---|
| | Hu BChE/PLL-g-PEO(2) | Hor BChE/PLL-g-PEO(2) |
| BChE alone | 353 | 923 |
| 1.2 | 347 | 840 |
| 2.3 | 357 | 867 |
| 3.4 | 353 | 890 |
| 6.2 | 363 | 947 |
| 8.2 | 373 | 930 |
| 10.3 | 390 | 913 |
| 12.1 | 420 | 933 |

The multimolecular core-shell structure of the block ionomer complexes can be reinforced by formation of cross-links between the polymer chains. The resulting cross-linked complexes are, in essence, nanoscale single molecules that are stable upon dilution and can withstand environmental challenges such as changes in pH, ionic strength, solvent composition and shear forces without structural deterioration. Therefore, to further increase the stability of the BChE/block ionomer complexes the cross-links were introduced in the complex structure. Glutaraldehyde (GA), an amine-reactive homofunctional cross-linker was used in these studies. Cross-linkage occurs due to formation of imines (Schiff base) between the aldehyde groups of GA and the primary amino groups of the both protein and polylysine segments of the block ionomer.

To introduce cross-linking to the complexes, Hu BChE/PLL-g-PEO(2) complexes (Z+/-=1.2, 0.15 mg/ml on BChE base) in 10 mM phosphate buffer (pH 7.4) were treated with a 0.25% solution of GA in water. The amount of GA was calculated on the basis of the targeted cross-linking ratio (85%) defined as the total amount of aldehyde groups in the GA solution versus total number of Lys residues in PLL-g-PEO copolymer. The cross-linked solutions of the complexes were kept for 5 hours at room temperature. The stability of the cross-linked complexes against dilution was evaluated using the Karnovsky & Roots method. Cross-linked complex was diluted in 1000, 5000, and 250 timed, respectively. Hu BChE and original non-cross linked complex diluted to the same extent were used as controls. A gel electrophoresis pattern is presented in Figure 4B. No BChE bands were observed in the lanes corresponding to cross-linked Hu BChE/PLL-g-PEO(2) complexes up to 1000-fold dilution. In contrast, dilution of complexes-precursors resulted in complete dissociation and release of free BchE. These data suggest that the stability of block ionomer complexes entrapping BchE in the core can be significantly increased by introducing cross-linking in the core of the complexes.

Enzymatic activity of Hu BChE incorporated into the cross-linked complexes was further assessed using butyrylthiocholine iodide as a substrate. It is a small enough molecule to penetrate into the cross-linked complexes to react with entrapped enzyme. The data are presented in Table 5. These data indicated that cross-linking of BChE/PLL-g-PEO complexes resulted in the loss of enzymatic activity of BChE entrapped into the complex (e.g., 75% decrease in the initial specific activity of BChE was observed). Overall, cross-linking of the core of BChE/PLL-g-PEO complexes results in sufficient resistance of the resultant BChE/PLL-g-PEO complexes to dilution.

**Table 5**

| Systems | Activity(units/mg) |
|---|---|
| Hu BChE | 320 |
| Hu BChE/PLL-g-PEO(2) (Z_{+/-}= 1.2) | 313 |
| Cross-linked Hu BChE/PLL-g-PEO(2) (Z_{+/-}= 1.2) | 76 |

To introduce various cross-linking to the complexes, Hu BChE/PLL-g-PEO(2) complexes (Z+/-=1.2, 0.15 mg/ml on BChE base) in 10 mM phosphate buffer (pH 7.4) were treated with a solution of GA in water. 3 µL of GA solutions with various concentrations were added to 120 µL of the complex solution as presented in Table 6. The amount of GA was calculated on the basis of the targeted cross-linking ratio defined as the total amount of aldehyde groups in the GA solution versus total number of Lys residues in PLL-g-PEO copolymer. It is noteworthy that the extent of targeted cross-linking represents the maximum theoretical amount of cross-linking that can take place, rather than the precise extent of amidation, which is expected to be lower. The targeted degree of cross-linking was varied from 10% to 100%. Mixtures were kept for 5 hours at room temperature.

**Table 6 - * Amount of Lys residues was 1.9 x 10⁻⁵ mmol.**

| Targeted cross-linking ratio (%) | C_{GA} (mg/ml) | GA (mmol)* |
|---|---|---|
| 100 | 0.25 | 1.9 x 10⁻⁵ |
| 85 | 0.25 | 1.6 x 10⁻⁵ |
| 40 | 0.125 | 3.75 x 10⁻⁶ |
| 20 | 0.062 | 1.9 x 10⁻⁶ |
| 10 | 0.031 | 9.4 x 10⁻⁷ |

The stability of the cross-linked complexes against dilution was evaluated using the Karnovsky & Roots method. Cross-linked complexes were diluted 1:1000, 1:500, and 1:250. Hu BChE and original non-cross linked complexes diluted to the same extent were used as controls. Representative gel electrophoresis patterns for the complexes with various cross-linking ratio (85%, 40%, and 20%) are shown in Figures 5A-5C. The complexes prepared at targeted cross-linking ratio of 85% and 40% were stable and did not dissociate upon dilution up to 1000 times. No BChE bands were observed in the lanes corresponding to cross-linked Hu BChE/PLL-g-PEO(2) complexes with targeted cross-linking of 85% (Figure 5A) and 40% (Figure 5B). Dilution of complexes-precursors resulted in complete dissociation and release of free BchE (lanes B). The complexes prepared at a targeted cross-linking ratio of 20% partial dissociated at higher dilutions (Figure 5C). Indeed, a band corresponding to free BChE was observed in the lanes corresponding to cross-linked complexes at 250-fold dilution.

Figure 6 presents the gel electrophoresis pattern observed for the BChE/ PLL-g-PEO(2) complexes (Z+/-= 1.2) prepared at various cross-linking ratio and diluted 500 times. The band of free BChE appeared in the lanes corresponding to the cross-linked complexes with cross-linking ratio of 30% and lower. These data suggest that cross-linking is preferably introduced into the BChE/ PLL-g-PEO(2) complexes at a targeted cross-linking ratio of at least 40% to prevent the degradation of complexes upon dilution.

Molecular mass (Mw) of cross-linked Hu BChE/PLL-g-PEO(2) complexes was measured via sedimentation equilibrium analysis. All measurements were made at 20°C at rotor speed of 6000 rpm during sedimentation time of 24 hours. Resulting sedimentation equilibrium pattern were recorded with an UV absorbance optical system. An average protein partial specific volume of 0.73 cm³/g was used for calculation of molecular weights from measured sedimentation equilibria. The calculated molecular masses are presented in Table 7. The molecular mass of the cross-linked complexes are comparable with those for complexes-precursor. These data suggest that cross-linking reactions proceeded within individual complex particles and did not result in inter-particle cross-linking and aggregation of complexes.

**Table 7**

| Sample | M_{w} | Variance | Number of polymer chains per BChE tetramer |
|---|---|---|---|
| Hu BChE alone | 364,215 | 1.14 x 10⁻⁵ | - |
| Hu BChE/PLL-g-PEO(2), (Z+/-=1.2) | 420,489 | 1.18 x 10⁻⁵ | 2.25 |
| Hu BChE/PLL-g-PEO(2), (Z+/-=1.2), 40% targeted cross-linking ratio | 450,509 | 1.0 x 10⁻⁵ | 3.12 |

Enzymatic activity of Hu BChE incorporated into the cross-linked complexes was further assessed using butyrylthiocholine iodide as a substrate. The data are presented in Table 8. These data indicated that cross-linking of BChE/PLL-g-PEO complexes affected the activity of BChE incorporated into the core of complex. Increasing the cross-linking ratio resulted in the loss of enzyme activity. For example, a 75% decrease in the initial specific activity of BChE was observed at targeted cross-linking ratio of 85% and no activity was determined at 100% of cross-linking. In contrast at the cross-linking ratio of 40%, the observed decrease in activity was rather small (20%). In conclusion, chemical cross-linking of the core of BChE/PLL-g-PEO complexes represent an effective tool to tune the stability of the complexes against dilution while preserving an activity of protein incorporated into ionic core of the complexes.

**Table 8**

| System | Targeted cross-linking ratio (%) | Activity(units/mg) |
|---|---|---|
| Hu BChE | 0 | 320 |
| Hu BChE/PLL-g-PEO(2) (Z_{+/-}= 1.2) | 0 | 313 |
| Cross-linked Hu BChE/PLL-g-PEO(2) (Z_{+/-}= 1.2) | 100 | 0 |
| | 85 | 76 |
| | 40 | 253 |
| | 20 | 248 |
| | 10 | 257 |

The in vivo migration and localization of BChE delivered by means of polymer complex was evaluated in butyrylcholinesterase nullizygote (BChE-/-) mice using optical imaging. BChE-/- knockout mice were produced by gene-targeted deletion of a portion of the BCHE gene (accession number M99492; Li et. al. (2008) J. Pharm. Exp. Ther., 324:1146-1154). Near-infra-red fluorescent probe IRDye®800CW (Li-cor, Lincoln, NE) was used to label Hor BChE. The degree of labeling was calculated to be one dye molecule per protein tetramer. To prepare complexes containing labeled Hor BChE (Hor BChE/IRDye), 16 µL solution of Hor BChE/IRDye were mixed with 57 µL of PLL-g-PEO(2)solution (10 mg/ml) and 8 µL of 10X PBS buffer (0.1 M phosphate buffer, C(NaCl)=1.4 M, pH 7.4). The resulted complexes were further cross-linked using glutaraldehyde. The amount of added glutaraldhyde was calculated on the basis of 40% of targeted degree of cross-linking. Mixture was kept for 5 hours at room temperature. The cross-linked Hor BChE/IRDye/ PLL-g-PEO(2)complex was stable against dilution as was confirmed by Karnovsky & Roots method. An overall observed decrease in enzymatic activity of Hor BChE/IRDye incorporated into the polymer complex due to cross-linking procedure was approximately 35%.

Prior to imaging, the hair on the animal's ventral and dorsal sections was removed using Nair cream. Mice were kept on a special purified diet to reduce the interfering fluorescence signals in the stomach and intestine that are induced by the standard animal food. Two routes of injection, intrathecal (IT) and intramuscular (IM), were used. Animals were anesthetized and then dosed with labeled protein or Hor BChE/IRDye incorporated into the cross-linked complex. Using the IVIS 200 imager the in vivo fluorescence of Hor BChE/IRDye was tracked over a 48-hour period. Accumulation of Hor BChE/IRDye incorporated in polymer complex was observed in the brain in 2.5 hours post IT injection of the complex. Fluorescence signal corresponding to Hor BChE/IRDye was also detected in the brain of the mouse in 48 hours after intramuscular injection of the complex.

To determine the final activity of the delivered BChE enzymes in the brain, mice were euthanized and brain tissues are excised for the analysis. Brain-associated BChE activity was determined using Ellman assay (Duysen, et al. (2001) J. Pharm. Exp. Ther. 299:528-535). Units of activity were defined as micromoles of butyrylthiocholine hydrolyzed per minute at pH 7.0, 25°C, and. The data are presented in Table 9.

**Table 9: These data demonstrate that BChE enzyme delivered within polymer complexes is accumulated and retained its activity in the brain tissue of the tested animals.**

| Treatment | Dose (BChE,mg) | Activity (units/g of tissue) |
|---|---|---|
| BChE/IRDye alone, **IT** | 0.05 | 0.09 |
| *cl*BChE/IRDye/PLL-*g*-PEO(2), **IT** | 0.019 | 0.07 |
| *cl*BChE/IRDye/PLL-*g*-PEO(2), **IM** | 0.075 | 0.01 |

### EXAMPLE 3

The following procedure was used to study biodistribution of CuZnSOD-polyion complex in living animals.

*Protein labeling.* CuZn superoxide dismutase (CuZnSOD; 2 mg) was dissolved in 1 ml Phosphate Buffered Saline (PBS: 0.1 M potassium phosphate, 1.5 M NaCl, pH 7.4) at room temperature. 100 µl of 1 M potassium phosphate buffer (K₂H₂PO₄) was added to the solution to raise the pH to 8.5. The obtained solution was transferred to the vial with reactive dye, Alexa 680 (Molecular Probes, Inc., Eugene, OR, cat # A-20172), and incubated with stirring for one hour at room temperature.

*Purification of labeled CuZnSOD.* A reaction mixture (1 ml) was applied on a column of Sephadex G-25 (0.5 x 26 cm) and phosphate buffer (10mM, pH 7.4) as an elution buffer. Two colored bands represented the separation of the labeled protein from unconjugated dye. The first colored band (light blue) was collected in about 30 minutes in eight fractions (150 µl each fraction). The protein concentration determined using the Pierce BCA assay was 0.75 mg/ml. The solution of labeled protein was lyophilized and stored at -20°C.

*Preparation of protein-incorporated polyion complexes.* To obtain CuZnSOD-polyion complex with +/- charge ratio (Z) = 2:1, 500 µl solution of Alexa 680-labeled CuZnSOD (1 mg/ml) in physiological buffer was added drop-wise to 830 µl solution of poly(ethyleneimine) (PEI) and poly(ethylene glycol) (PEG) block-copolymer (PEI-PEG, 2 mg/ml) with stirring. The +/- charge ratio (Z) was calculated by dividing the amount of amino groups of PEI-PEG protonated at pH 7.4 by the total amount of Gln and Asp in CuZnSOD. The obtained CuZnSOD-polyion complex solution was incubated at least 1 hour before further use.

*Visualization of CuZnSOD-polyion complex biodistribution in mice.* Prior to the experiment, BALB/C female mice were anesthetized with pentobarbital i.p. injections at the dose of 30-40mg/kg body weight, shaved and depilated (to reduce fluorescence blocking by hair). The mice were kept on liquid diet for 72 hours (to eliminate autofluorescence in stomach and intestine from solid food). The mice were tail vein-injected with Alexa-680 labeled CuZnSOD-polyion complexes. Then, the mice were anesthetized with a 1.5% isoflurane mixture with 66% nitrous oxide and the remainder oxygen and placed into imaging camera. The biodistribution of CuZnSOD-polyion complexes was determined by measuring the *in vivo* fluorescence of Alexa-680 as detected by an IVIS 200 Series Imaging Gas Anasthesia System. Alexa 680-labeled CuZnSOD-polyion complexes started to accumulate in the brain 1 hour after IV injection, peaked at 7 hours post-injection, and remained elevated for at least 24 hours post-injection (Figure 7). These data indicate that peripherally administered CuZnSOD-polyion complexes is localized to the brain.

### EXAMPLE 4

PLL-PEO copolymers having a block architecture were used to incorporate BChE in block copolymer complexes. Poly-L-lysine-graft-poly(ethylene oxide) (PLL-b-PEO) was synthesized (see, e.g., Harada et al. (1995) Macromolecules 28:5294). α-methoxy-ω-amino-poly(ethylene glycol) with a molecular weight of 5,600 g/mol and rather narrow molecular weight distribution of 1.27 (Biotech GmbH, Germany) was used as a macroinitiator for the synthesis of block copolymer. PLL-b-PEO was characterized by ¹H NMR spectroscopy using D₂O as a solvent on a Varian 500 MHz spectrometer. The length of PLL segment was calculated to be 25. An estimated molecular mass of PLL-g-PEO is ca. 24,000 g/mol. This polymer was designated as PLL-b-PEO. The peak intensity ratio of methylene protons of PEO (OCH₂CH₂: δ = 3.62 ppm) and ε-methylene protons of PLL ((CH₂)₃CH₂NH₃: δ = 2.9 ppm) was measured to calculate the degree of polymerization value for PLL segment which was determined to be 36. An estimated molecular mass of PLL-b-PEO is ca. 10,200 g/mol. This polymer was designated as PLL-b-PEO.

Reverse titration was carried out to determine the concentration of amino group in PLL-b-PEO solution. The concentration of amino groups in 5 mg/ml solution of PLL-b-PEO was calculated to be 6.1 mM.

Both samples of human BChE (Hu BChE) and BChE from equine serum (Hor BChE) were used to prepare complexes with PLL-b-PEO. Complexes were prepared by simple mixing of buffered solutions (phosphate buffer, 10 mM, pH 7.4) of the block copolymer and protein components at various compositions of mixture and presented in Table 10. The compositions of the BChE/PLL-b-PEO mixtures were expressed in terms of total amount of carboxylic groups (Glu, Asp, and sialic acid) in protein and calculated as a ratio of concentration of amino group in PLL-b-PEO to the total concentration of carboxylic groups in protein (Z+/-).

**Table 10**

| Sample | |
|---|---|
| (Hu BChE/PLL-*b-*PEO or Hor BChE/PLL-*b*-PEO) | Z_{+/-} |
| 1 | 0.5 |
| 2 | 1.0 |
| 3 | 2.0 |
| 4 | 3.0 |

The extent of incorporation of BChE into block ionomer complexes was monitored using non-denaturating PAGE. Figures 8A and 8B present the gel electrophoresis patterns observed for Hu BChE/PLL-b-PEO and Hor BChE/PLL-b-PEO mixtures, respectively. In both cases BChE bands intensity decreased as the amount of block copolymer in the mixture was increased. This demonstrated that the PLL-b-PEO block copolymer was binding to the BChE and neutralizing its charge. Practically complete retardation of complex migration in the gels was observed in the vicinity of Z+/- = 2.0 for both Hu BChE/PLL-b-PEO and Hor BChE/PLL-b-PEO mixtures. It is noteworthy that an incorporation of BChE from equine serum (Hor BChE) into the block ionomer complexes using PLL-PEO copolymers of graft architecture (PLL-g-PEO(2) or PLL-g-PEO(7)) required the presence of the excess of the copolymer in the mixtures (Z+/-=6.2).

The complexes of BChE of both types and PLL-b-PEO were further characterized by dynamic light scattering. The data for all types of complexes studies are summarized in Table 11. Particles of slightly larger size than protein alone were detected in BChE/block copolymer mixtures.

**Table 11**

| Sample | Z_{+/-} | Diameter. nm |
|---|---|---|
| Hu BChE | - | 13.30 |
| Hu BChE/PLL-*b*-PEO | 1.0 | 14.63 |
| Hu BChE/PLL-*b*-PEO | 2.0 | 14.35 |
| Hor BChE | - | 12.20 |
| Hor BChE/PLL-*b*-PEO | 1.0 | 13.92 |
| Hor BChE/PLL-*b*-PEO | 2.0 | 13.20 |

The effect of cross-linking of the core of BChE/PLL-b-PEO complexes on stability of the complexes was further elucidated. Glutaraldehyde (GA), an amine-reactive homofunctional cross-linker was used in these studies. To introduce cross-linking to the complexes, both Hu BChE/PLL-b-PEO and Hor BChE/PLL-b-PEO complexes (Z+/-=1.0, 0.15 mg/ml on BChE base) in 10 mM phosphate buffer (pH 7.4) were treated with a 0.008% solution of GA in water. The amount of GA was calculated on the basis of the targeted cross-linking ratio (40%) defined as the total amount of aldehyde groups in the GA solution versus total number of Lys residues in PLL-b-PEO copolymer. The solutions of the complexes with added cross-linker were kept for 5 hours at room temperature. The stability of the cross-linked complexes against dilution was evaluated using the Karnovsky & Roots method. Cross-linked complexes were diluted 500 times. BChE samples and original non-cross linked complexes diluted to the same extent were used as controls. The gel electrophoresis pattern is presented in Figure 9A. These data indicate that BChE/PLL-b-PEO complexes prepared at a composition of Z+/-=1.0 and at targeted cross-linking ratio of 40% were unable to resist dilution that led to their dissociation. A band corresponding to free BChE was observed in all lanes corresponding to cross-linked complexes (lanes C and F of Fig. 9A, respectively) and their non cross-linked precursors (lanes B and E of Fig. 9A, respectively).

In another set of experiments, Hu BChE/PLL-b-PEO and Hor BChE/PLL-b-PEO complexes prepared at Z+/-=2.0 (0.15 mg/ml on BChE base) were treated with a 0.016% solution of GA to achieve a targeted degree of cross-linking of 40%. Cross-linked complexes were diluted 500 times. BChE samples and original non-cross linked complexes diluted to the same extent were used as controls. The gel electrophoresis pattern is presented in Figure 9B. As it seen in Figure 9B, no BChE bands were observed in the lanes C and F corresponding to cross-linked Hu BChE/PLL-b-PEO and Hor BChE/PLL-b-PEO complexes with Z+/-=2.0, respectively. In contrast, dilution of complexes-precursors resulted in complete dissociation and release of free BchE (lanes B and E of Fig. 9B). Therefore, it appears that a small excess of block copolymer in the BChE/PLL-b-PEO complexes might be necessary for successful cross-linking of the complex core.

Enzymatic activity of BChE incorporated into the non cross-linked and cross-linked BChE/PLL-b-PEO complexes was further assessed using butyrylthiocholine iodide as a substrate. The data are presented in Table 12. Practically no changes in enzymatic activity of Hor BChE incorporated in cross-linked Hor BChE/PLL-b-PEO complex (Z+/-=2) were found. Furthermore, no decrease of enzymatic activity of Hu BchE was observed in the case of cross-linked Hu BChE/PLL-b-PEO complexes as compared to BChE activity measured in the solutions of non cross-linked complexes.

**Table 12**

| Systems | Activity(units/mg) |
|---|---|
| Hu BChE | 437 |
| Hu BChE/PLL-*b*-PEO, Z_{+/-}= 2 | 260 |
| Cross-linked Hu BChE/PLL-*b*-PEO, Z_{+/-}= 2 | 260 |
| Hor BChE | 573 |
| Hor BChE/PLL-*b*-PEO, Z_{+/-}= 2 | 547 |
| Cross-linked Hor BChE/PLL-*b*-PEO, Z_{+/-}= 2 | 610 |

### EXAMPLE 5

Entry into the brain occurs as a consequence of the establishment of a chemokine gradient induced through neuroinflammatory responses (Kadiu et al. (2005) Neurotox. Res., 8:25-50; Gorantla et al. (2006) J. Leukocyte Biol., 80:1165-1174). Thus, a PD-like model system was developed for testing the utility of cell-based delivery. First, divergent inflammatory cues were used to stimulate ROS production from microglia and included nitrated alpha synuclein (N-α-syn), thought to be released extracellularly in PD and elicit immune activation (Gendelman, H. (2006) Neurotoxicology 27:1162; Mosley et al. (2006) Clin. Neurosci. Res., 6:261-281; El-Agnaf et al. (2003) FASEB J., 17:1945-7). Second, 1-methyl-4-phenyl-1,2,3,6-tetrahydropyridine (MPTP)-induced inflammation served as a gradient for BMM ingress into the brain. It is well-documented that following inflammatory cues, leukocytes are recruited to the brain through diapedesis and chemotaxis (Anthony et al. (1997) Brain 120:435-44; Anthony et al. (2001) Prog. Brain Res., 132:507-24; Blamire et al. (2000) J. Neurosci., 20:8153-9; Persidsky et al. (1999) Am. J. Pathol., 155:1599-611; Kuby, J. (1994) Immunology; Freeman, WH. and Co., New York). Monocyte-macrophages can migrate across the brain paracellular spaces crossing junctional complexes of brain endothelial cells (Pawlowski et al. (1988) J. Exp. Med., 168:1865-82; Lossinsky et al. (2004) Histol. Histopathol., 19:535-64). Their combat arsenal consists of engulfing foreign particles and liberating engulfed substances by exocytosis. All together, these features make it possible to exploit macrophages as carriers to affect neuroinflammatory processes (Daleke et al. (1990) Biochim. Biophys. Acta 1024:352-66; Lee et al. (1992) Biochim. Biophys. Acta 1103:185-97; Nishikawa et al. (1990) J. Biol. Chem., 265:5226-31; Fujiwara et al. (1996) Biochim. Biophys. Acta 1278:59-67).

Here, BMM was used as a vehicle for carriage of therapeutic concentrations of catalase to the brain. A major obstacle for success in this approach is that macrophages efficiently disintegrate engulfed particles (Fujiwara et al. (1996) Biochim. Biophys. Acta 1278:59-67). Therefore, it is crucial to protect the activity of the enzyme inside of the cell carrier. Incorporation into polymeric nanocarries (nanospheres, liposomes, micelles, nanoparticles) can provide such protection (Aoki et al. (2004) Int. J. Hypertherm., 20:595-605; Calvo et al. (2001) Pharm. Res., 18:1157-1166; Gref et al.; (1994) Science 263:1600-1603; Harada et al. (1999) Science 283:65-7; Jaturanpinyo (2004) Bioconjugate Chem., 15:344-8; Kabanov et al. (2002) J. Controlled Release 82:189-212; Kwon, G.S. (2003) Crit. ReV. Ther. Drug Carrier Syst., 20:357-403; Mora et al. (2002) Pharm. Res., 19:1430-8; Rousseau et al. (1999) Exp. Brain Res., 125:255-64; Torchilin, V.P. (2000) Eur. J. Pharm. Sci., 11:S81-91; Vinogradov et al. (2004) Bioconjugate Chem., 15:50-60). Previous work has demonstrated that use of interpolyelectrolyte complexes can immobilize enzymes (Kabanov et al. (1977) Mol. Biol. (Russian), 11:582-596; Kabanov, V. (1994) Polym. Sci., 36:183-197; Kabanov et al. (2004) J. Phys. Chem. B, 108:1485-1490). The enzyme polyelectrolyte complexes can be prepared at the nanoscale by self-assembly of enzymes with oppositely charged block polyelectrolytes containing ionic and nonionic water soluble blocks (Harada et al. (2001) J. Controlled Release 72:85-91; Harada et al. (2003) J. Am. Chem. Soc., 125:15306-7). The resulting nanoparticles contain a core of protein-polyelectrolyte complex surrounded by a shell of water soluble nonionic polymer such as polyethylene glycol (PEG). In the current work, catalase was immobilized by reacting it with a cationic block copolymer, polyethyleneimine-poly(ethylene glycol) (PEI-PEG), previously used for delivery of polynucleotides (Vinogradov et al. (1998) Bioconjugate Chem., 9:805-812). The resulting block ionomer complexes of catalase are taken up by BMM. Evidence is presented here that such modification protects catalase against degradation in BMM, that BMM release polypeptide-polyion complexes in the external medium for at least 4-5 days, and that BMM can carry polypeptide-polyion complexes to the brain, such as in the MPTP model of PD.

### Materials and Methods

*Materials.* Same as in Example 1. *BMM.* Bone marrow cells extracted from murine femurs (C57BL/6, female mice) as described (Dou et al. (2006) Blood 108:2827-35) were cultured for 10 days in the media supplemented with 1000 U/mL macrophage colony-stimulating factor (MCSF) (Wyeth Pharmaceutical, Cambridge, MA). The purity of monocyte culture was determined by flow cytometry using FACSCalibur (BD Biosciences, San Jose, CA).

*Microglia.* Brains from C57BL/6 neonates (1-3 days old) were removed, washed with ice-cold HBSS, and mashed into small pieces. Supernatant was replaced for 2.5% trypsin and DNAse solution (1 mg/mL) and incubated for 30 minutes at 37°C, and then 1 mL of ice cold FBS with 10 mL HBSS was added. The mixture was centrifuged (5 minutes, 1500 rpm, 4°C), and complete media with MCSF was added to the pellet. The cells were cultured until maturation (typically 10 days).

*MPTP.* Same as in Example 1.

*PEI-PEG Conjugates.* Same as Example 1.

*Block Ionomer Complexes.* Same as Example 1.

*Electrophoretic* Retention. Same as Example 1.

*Light Scattering Measurements.* Same as Example 1.

*TEM.* Same as Example 1.

*Catalase and Catalase Activity.* Same as Example 1. *Labeling Catalase with Alexa Fluor 594 and Rhodamine Isothiocyanate (RITC).* For loading and release studies, the enzyme was labeled with Alexa Fluor 594 Protein Labeling Kit (A10239, Molecular probes, Inc., Eugene, OR) according to the manufacturers protocol. For confocal microscopy studies, catalase was labeled with RITC. Briefly, catalase was dissolved in 0.1 M sodium carbonate buffer, pH 8.5 (1 mg/mL), and treated with RITC (10 mg/mL) in DMSO for 2 hours at room temperature. Labeled catalase was purified from low molecular weight residuals by gel filtration on a Sephadex G-25 column (1 x 20 cm) in PBS at elution rate 0.5 mL min⁻¹ and lyophilized.

*Accumulation and Release of Polypeptide-Polyion Complexes in BMM.* BMM grown on 24-well plates (2.5 x 10⁶ cells/plate) (Batrakova et al. (1998) Pharm. Res., 15:1525-1532; Batrakova et al. (2005) Bioconjugate Chem., 16:793-802) were preincubated with assay buffer (122 mM NaCl, 25 mM NaHCO₃, 10 mM glucose, 3 mM KCl, 1.2 mM MgSO₄, 0.4 mM K₂HPO₄, 1.4 mM CaCl₂, and 10 mM HEPES) for 20 minutes. Following preincubation, the cells were treated with the Alexa-Fluor 594 labeled enzyme (0.7 mg/mL) in assay buffer alone or polypeptide-polyion complexes for various time points. After incubation, the cells were washed three times with ice-cold PBS and solubilized in Triton X 100 (1%). For measures of polypeptide-polyion complexes released from BMM, loaded BMM were incubated with fresh media at various time points. Fluorescence in each sample was measured by a Shimadzu RF5000 fluorescent spectrophotometer (λₑₓ) 580 nm, λₑₘ) 617 nm). The amount of polypeptide-polyion complexes was normalized for protein content and expressed in µg of enzyme per mg of the protein for loading experiments and µg enzyme per mL media as mean ± SEM (n = 4).

*Intracellular Localization of Polypeptide-Polyion Complexes.* Monocytes grown in the chamber slides (Kabanov et al. (1995) Bioconjugate Chem., 6:639-643) were exposed to RITC-labeled polypeptide-polyion complexes (Z = 1) for 24 hours at 37°C. Following incubation, the cells were fixed in 4% paraformaldehyde and stained with F-actin-specific Oregon Green 488 phalloidin and a nuclear stain, ToPro-3 (Molecular Probes, Inc., Eugene, OR). Labeled cells were examined by a confocal fluorescence microscopic system ACAS-570 (Meridian Instruments, Okimos, MI) with argon ion laser (excitation wavelength, 488 nm) and corresponding filter set. Digital images were obtained using the CCD camera (Photometrics, Tuscon, AZ) and Adobe Photoshop software.

*Antioxidant Activity Measures.* Mature mouse BMM were loaded with the enzyme alone or enzyme-polyion complexes (Z = 1) for 1 hour and washed with PBS, and fresh media was added to the cells. Following various time intervals, the media was collected and antioxidant activity of the enzyme released from BMM was assayed by the rate of hydrogen peroxide decomposition.

*Ampex Red Dye Fluorescence Assay.* Murine microglial cells seeded in 96-well plates (0.1 x 10⁶ cells/well) were either stimulated with tumor necrosis factor alpha (TNF-α) (200 ng/ mL) for 48 hours or with nitrated alpha-synuclein (N-α-syn) (0.5 µM) to induce ROS production. In parallel, BMM grown in 24-well plates were loaded with "naked" catalase (1 mg/mL) or catalase-polyion complexes for 1 hour and then incubated with Krebs-Ringer buffer (145 mM NaCl, 4.86 mM KCl, 5.5 mM glucose, 5.7 mM NaH₂PO₄, 0.54 mM CaCl₂, 1.22 mM MgCl₂, pH 7.4) for 2 hours to collect catalase released from the cells into the supernatant. Following incubation, the supernatants collected from BMM loaded with "naked" catalase or catalase-polyion complex were supplemented with Ampex Red Dye stock solution (10 U/mL HRP, 10 mM Ampex Red). For N-α-syn stimulation of microglia, supernatants were also supplemented with 0.5 µM aggregated N-α-syn. Obtained solutions were added to the activated microglial cells, and the decomposition of ROS by "naked" catalase or catalase-polyion complex was measured by fluorescence at λₑₓ = 563 nm, λₑₘ = 587 nm. The effect of the supernatants collected from nonloaded BMM or loaded with PEI-PEG alone on ROS decomposition was evaluated in comparison to the control experiments.

*¹²⁵I-Labeling of Catalase Polypeptide-Polyion Complex.* Same as Example 1. ¹²⁵I-labeled catalase (400 µCi/mL, 0.7 mg/mL) was supplemented with PEI-PEG block copolymer (Z = 1) and loaded into mature monocytes (80 x 10⁶ BMM in 1 mL of medium) for 2 hours at 37°C. After incubation, the loaded monocytes were washed three times with ice-cold PBS.

*Statistical Analysis.* Same as Example 1.

### Results

The manufacture of the polypeptide-polyion complexes is described hereinabove in Example 1. Initially, using the sulforhodamine-B (SRB) cell viability assay, it was demonstrated that polypeptide-polyion complexes (as well as catalase or copolymer alone) did not induce BMM cytotoxicity over a wide range of concentrations (0.03 to 1000 µg catalase per mL; Figure 10). The accumulation kinetics suggested a rapid uptake of both free catalase and polypeptide-polyion complex in BMM (Figure 11A). Notably the free enzyme was taken up in BMM almost twice as fast as the polypeptide-polyion complex. At the 60 minute time point, the loading of BMM with polypeptide-polyion complex was ca. 30 µg catalase/10⁶ cells. The uptake of the polypeptide-polyion complex at the 60 minute time point decreased as the charge ratio increased (Figure 11B), which may be due to the effect of the PEG corona. The confocal microscopy data suggested vesicular and/or cytoplasmic localization of RITC-labeled catalase administered to BBM in polypeptide-polyion complex (Figure 11C).

Mature BMM were preloaded with Alexa Fluor 594-labeled catalase-polyion complex (60 minutes) and then cultured in the fresh media for different time intervals. The loaded BMM released catalase in the external media for at least 4-5 days (Figure 12A). During the same period, the amount of the enzyme associated with the cells was proportionally decreased. Exposure of polypeptide-polyion complex -loaded BMM to 10 µM phorbol myristate acetate (PMA), a potent activator of the protein kinase C pathway and ROS generation (Chang et al. (1993) Immunology 80:360-366), enhanced enzyme release in the media by ca. 50% (Figure 12B). This suggested that release of polypeptide-polyion complex from BMM may be dependent on cell activation.

BBM loaded with "naked" catalase or catalase-polyion complex were placed in a fresh media, and the activity of the enzyme released in the media was determined at different incubation time intervals. Contrary to BMM loaded with free catalase that was practically inactive after the release, the catalase-polyion complex-loaded cells released active enzyme for at least 24 hours (Figure 13A). The maximal activity of the released enzyme was observed for BMM loaded with catalase-polyion complex prepared at the stoichiometric ratio, Z = 1 (Figure 13B). All together, this indicates that incorporation of catalase in a block ionomer complex with PEI-PEG results in protection and sustained release of active catalase from BMM.

To assess the antioxidant capacity of the catalase nanoformulations on microglial ROS production, BMM loaded with "naked" catalase or catalase-polyion complex were incubated for 2 hours in Krebs-Ringer buffer, and the reluctant supernatant was then collected and added to TNF-α (200 ng/mL)-stimulated microglial cells. The catalase in the supernatants collected from the catalase- or catalase-polyion complex-loaded BMM decomposed hydrogen peroxide by microglia (Figure 14A). A greater effect was observed by catalase-polyion complex, which was consistent with its ability to preserve enzyme activity in carrier cells. Furthermore the supernatants collected from unloaded BMM (Figure 14B) or from BMM loaded with PEI-PEG alone (Fig. 14C) had little, if any, effect on the hydrogen peroxide level. To determine whether these findings could be reproduced in microglia activated by stimuli typically found in PD, cells were stimualted with 0.5 µM N-α-syn. Aggregated N-α-syn present as cytoplasmic bodies in PD are released following the death of dopaminergic neurons and are a major component of Lewy bodies (Zhang et al. (2005) FASEB J., 19:533-42). These aggregated proteins are hypothesized to serve as a stimulus for microglial activation (Gendelman, H. (2006) Neurotoxicology 27:1162; Thomas et al. (2007) J. Neurochem. 100:503-19). Once again, the level of hydrogen peroxide was significantly reduced with the addition of supernatants from catalase-polyion complex loaded BMM (Figure 14D). All together this study suggests that catalase-polyion complex released from BMM can attenuate oxidative stress resulting from activation of microglia. Indeed, catalase-polyion complex released from BMM decreased amount of H₂O₂ significantly grater than "naked" catalase, thereby indicating that the polyion complexes efficiently preserves enzymatic activity of catalase in BMM.

To determine if BMM carrying catalase-polyion complex could reach brain subregions with active neuroinflammatory disease reflective of human PD, the MPTP model was used. Two groups of MPTP-intoxicated C57B1/6 mice were either injected intravenously with free polypeptide-polyion complex containing ¹²⁵I-labeled catalase or received adoptively transferred catalase-polyion complex -loaded BMM. Twenty four hours after injection there were significant increases in the radioactivity levels in spleen, liver, lung, kindney, and brain in the groups receiving adoptive transfer compared to groups treated with catalase-polyion complex alone (Figure 15). It is noteworthy that after the adoptive transfer about 0.6% of the injected dose was found in the brain which was twice what was found in animals injected with free catalase-polyion complex. All together these data provide evidence that adoptive transfer of enzyme-polyion complex loaded BMM can increase the delivery of the enzyme to the brain as well as other peripheral tissues known to be sites of macrophage tissue migration.

Efficient transport of therapeutic polypeptides to the brain is required for successful therapies for neurodegenerative and neuroinflammatory diseases. To this end, it was examined whether BMM could be used as vehicles for delivery of a potent antioxidant, catalase. Indeed, it has long been known that macrophages and microglia as well as other mononuclear phagocytes can endocytose colloidal nanomaterials, for example, liposomes or nanosuspensions, and subsequently carry and release the drug to site of tissue injury, infection, or disease (Dou et al. (2006) Blood 108:2827-35; Dou et al. (2007) Virology 358:148-158; Gorantla et al. (2006) J. Leukocyte Biol., 80:1165-1174; Daleke et al. (1990) Biochim. Biophys. Acta 1024:352-66; Jain et al. (2003) Int. J. Pharm., 261:43-55).

Moreover, the abilities of BMM to cross BBB was also investigated (Lawson et al. (1992) Neuroscience 48:405-15; Simard et al. (2004) FASEB J., 18:998-1000; Male et al. (2001) Prog. Brain Res., 132:81-93; Streit et al. (1999) Prog. Neurobiol., 57:563-81; Kokovay et al. (2005) Neurobiol. Dis., 19:471-8; Kurkowska-Jastrzebska et al. (1999) Acta Neurobiol. Exp. (Wars) 59:1-8; Kurkowska-Jastrzebska et al. (1999) Exp. Neurol., 156:50-61; Simard et al. (2006) Mol. Psychiatry 11:327-35). In particular, it was demonstrated that monocytes infiltrate the brain in the MPTP mouse model of PD (Kokovay et al. (2005) Neurobiol. Dis., 19:471-8; Kurkowska-Jastrzebska et al. (1999) Acta Neurobiol. Exp. (Wars) 59:1-8; Kurkowska-Jastrzebska et al. (1999) Exp. Neurol., 156:50-61). Indeed, MPTP toxicity stimulated transient and global increases in the rate of monocyte infiltration into the midbrain, stratum, septum, and hippocampus. In these prior studies, the maximal accumulation of the monocyte-macrophages in the brain was observed 1 day after the MPTP treatment. On the basis of these data, it appears that catalase-loaded monocytes adoptively transferred in MPTP-treated mice can deliver enzyme to regions of the brain most affected in PD including the substantia nigra and striatum.

To protect against catalase degradation inside the BMM, the protein was immobilized in the block ionomer complex with a cationic block copolymer, PEI-PEG. The resulting nanoparticles were ca. 60 to 100 nm in size and stable in physiological conditions (pH, ionic strength). The composition and structure of the catalase-polyion complexes was altered to achieve high loading in BMM and preserve catalase activity. Internalization of foreign particles, as well as the exocytotic secretion, is one of the most basic functions in macrophages (Stout et al. (1997) Front. Biosci., 2:d197-206). It has been demonstrated herein that BMM can accumulate a significant amount of polypeptide-polyion complex (ca. 30 µg catalase/10⁶ cells) in a relatively short time period (about 40-60 minutes), followed by its sustained release during 4-5 days into the external media. This also suggested that catalase-polyion complex-loaded cells after adoptive transfer may have sufficient time to reach the brain and release catalase. Moreover, it was reported (Schorlemmer et al. (1977) Clin. Exp. Immunol., 27:198-207; Allison et al. (1974) Symp. Soc. Exp. Biol., 419-46; Cardella et al. (1974) Nature 247:46-8) that exocytosis can be stimulated by activation of monocytes and macrophages. The above experiments show that release of polypeptide-polyion complex by BMM can be enhanced by stimulation with PMA. It is also demonstrated above that block ionomer complex protects the activity of catalase inside the host cells. Notably, the enzyme-polyion complex-loaded BMM released active enzyme in the media for at least 24 hours. Furthermore, the culture supernatants collected from polypeptide-polyion complex-loaded BMM had potent antioxidant effects in the assay for ROS produced by microglia activated with either N-α-syn or TNF-α. Thus, these cell culture models indicate that polypeptide-polyion complex-loaded BMM can mitigate oxidative stress associated with the neurodegenerative process. Finally, *in vivo* evidence that adoptive transfer of polypeptide-polyion complex-loaded BMM can increase delivery of labeled enzyme into the tissues including 2-fold increase in the amount of the enzyme in the brain in MPTP-treated mice is provided. Interestingly, considerable amount of the labeled enzyme was also found in the brain after injection of the polypeptide-polyion complex alone. It is possible that the polypeptide-polyion complex may be taken up by circulating monocytes, which then carry the enzyme to the brain.

### EXAMPLE 6

*Image Visualization and in Vivo Imaging System (IVIS) studies.* BALB/C mice were injected with MPTP (to induce PD-related neuroninflammation) and shaved (to reduce fluorescence blocking by hair). Alexa 680-labeled polypeptide-polyion complex (PEI-PEO; Z=1) was loaded into BMM, and then the monocytes were administered i.v. to MPTP-treated mice (50 mln/mouse). The mice were imaged using IVIS for various time intervals (Fig. 16). Significant amount of polypeptide-polyion complex was found in MPTP-intoxicated brain. Significantly, no fluorescence was detected in the brain of non-MPTP control mice indicating that BMM facilitated polypeptide-polyion complex delivery to the inflammation sites across the BBB.

*Histopathological evaluation of polypeptide-polyion complex-loaded BMM toxicity in vivo.* C57BL/6 healthy mice were injected with monocytes loaded with polypeptide-polyion complex (10 mln/mice) or PBS (control group). 48 hours later brain, liver, spleen, and kidney were collected at necropsy. Coded H&E stained organs sections were examined by light microscopy. No signs of apoptosis, BBB break-down, neuron-inflammatory response of neuronal cell death in the brain; macrovesicular steatosis and necrosis of hepatocytes; signs of cholestiasis in liver; or signs of acute tubular necrosis in kidneys were found.

*Neuroprotection of polypeptide-polyion complex loaded into BMM against MPTP-induced dopaminergic neuronal loss in mice.* To assess polypeptide-polyion complex neuroprotective effect, MPTP-intoxicated mice were injected i.v. with polypeptide-polyion complex-loaded BMM and levels of the brain neuronal metabolite N-acetyl aspartate (NAA) in the SNpc and stratum (the regions most affected in human disease) were monitored on day seven after the treatment. MPTP injections caused significant loss of NAA in SNpc and stratum of control mice (Fig. 17). In contrast, there was no reduction in NAA levels in MPTP-intoxicated mice treated with polypeptide-polyion complex loaded in BMM. In additional studies, the brains, particularly the SNpc and stratum, of mice intoxicated with MPTP and then intravenously administered BMM loaded with catalase-polyion complexes, were found to have reduced levels of inflammation as measured by astrocytosis to that of control mice levels after two days. The above indicates that catalase-polyion complex has a neuroprotective capacity during MPTP-induced dopaminergic neurodegeneration.

### Example 7

### Peripheral administration of CuZnSOD-polyion complex inhibits the acute blood pressure response of centrally administered AngII.

The CuZnSOD-polyion complex described in Example 3 was used to provide evidence that peripherally administered CuZnSOD-polyion complex is able to modulate AngII signaling in the brain. Specifically, the experiment examined effects of peripherally administered (intra-carotid) CuZnSOD-polyion complex on the acute increase in blood pressure induced by AngII (100 ng) given ICV. The ICV AngII-induced changes in mean arterial pressure (MAP) were recorded in rabbits 0, 1, 2, and 5 days following intra-carotid administration of CuZnSOD-polyion complex or free CuZnSOD. The change in MAP following ICV administered AngII was drastically reduced 1 and 2 days after CuZnSOD-polyion complex treatment compared to the response at Day 0 (Fig. 18). In contrast, treatment with free CuZnSOD protein, which is active but unable to pass through cell membranes, had no effect on the ICV AngII-induced blood pressure response (Fig. 18). These data indicate that CuZnSOD-polyion complex given peripherally is able to permeate AngII-sensitive neurons in the CNS and modulate central AngII-mediated cardiovascular responses. Indeed, methods of treating hypertension in a patient are described herein which
comprise the administration of a composition comprising
a) at least one complex comprising copper zinc superoxide dismutase (CuZnSOD) and a synthetic polymer comprising at least one charge opposite to the charge of the CuZnSOD, and b) at least one pharmaceutically acceptable carrier. The complex comprising CuZnSOD and a synthetic polymer comprising at least one charge opposite to the charge of the CuZnSOD may be contained within a cell, which is administered to a patient.

### EXAMPLE 8

Brain-derived neutrophic factor (BDNF) is a basic neurotrophic protein of molecular weight of 27.3 kDa with isoelectric point of 10.23. BDNF has a net positive charge (+ 9.5) at neutral pH (Philo et. al. (1994) J. Biol. Chem., 269:27840-27846). Therefore, an anionic block copolymer, PEO-b-poly(sodium methacrylate) (PEO-b-PMA) (pKa of carboxylic group is 5.2) was used to incorporate BDNF into the polyion complex. Complexes were prepared by simple mixing of buffered aqueous solutions of the block copolymer and protein components. The polymer/protein ratio in the mixtures was calculated by dividing the total calculated concentration of carboxylic groups of PEO-b-PMA by the concentration of total Lys and Arg residues in protein. Upon mixing, these systems remained transparent, and no precipitation was observed.

Herceptin (trastuzumab) is a humanized anti-human epidermal growth factor receptor 2 (HER2/c-erbB2) monoclonal antibody. Herceptin has been shown to be efficacious against primary and extracranial metastatic breast cancers that overexpress HER2. However, in patients with brain metastasis, the blood-brain barrier limits its use (Kinoshita et. al. (2006) PNAS, 103:11719-11723).

Herceptin is a basic protein of molecular weight of 145.5 kDa with isoelectric point of 8.45. Herceptin has a net positive charge (+ 12) at neutral pH. Anionic block copolymer, PEO-b-poly(sodium methacrylate) (PEO-b-PMA) (pKa of carboxylic group is 5.2) was used to incorporate Herceptin into the polyion complex. Complexes were prepared by simple mixing of buffered aqueous solutions of the block copolymer and protein components. The polymer/protein ratio in the mixtures was calculated by dividing the total calculated concentration of carboxylic groups of PEO-b-PMA by the concentration of total Lys and Arg residues in protein. Upon mixing, these systems remained transparent, and no precipitation was observed.

Leptin is a 18.7 kDa protein hormone that plays a key role in regulating energy intake and energy expenditure, including the regulation (decrease) of appetite and (increase) of metabolism. Leptin has an isoelectric point of 5.85 and a net negative charge (ca. -2) at physiological pH. Cationic block ionomer of graft architecture, poly-L-lysine-graft-poly(ethylene oxide), PLL-g-PEO(2), containing ca. 1.4 PEO chains grafted onto a PLL backbone, was used to prepare leptin-polyion complexes. Complexes were prepared by simple mixing of buffered aqueous solutions of the graft copolymer and protein components. The polymer/protein ratio in the mixtures was calculated by dividing the total concentration of amino groups of PLL-g-PEO(2) by the concentration of total Asp and Glu residues in protein. Upon mixing, these systems remained transparent, and no precipitation was observed.

### EXAMPLE 9

### Prevention of inflammation in MPTP-intoxicated mice by monocytes loaded with catalase polyion complexes.

For inducing pathological changes characterized for PD, male C7BL/6 recipient mice were administered at 18 mg freebase MPTP/kg body weight delivered in PBS by 4 intraperitoneal injections given every two hours (MPTP (Sigma Chemical Co., St. Louis, MO)). Control mice were injected with saline i.v. 18 hours later, half of MPTP-intoxicated mice were injected i.v. with monocytes loaded with catalase polyion complex (10mln/mouse) and another half was injected with saline i.v. The active phase of neuronal death and neuroinflammatory activities peak occurs at about 2 days after MPTP injection. Therefore, two days later, midbrain areas from naive, MPTP-intoxicated, and MPTP-intoxicated and then treated with catalase-loaded monocytes mice were isolated, brains were snap frozen, and embedded in OCT medium. Immunohistochemical analysis was performed in intact slices 30 µm thick fixed in 4% paraformhaldeyde for 24 hours and post-fixed in sucrose solution for 48 hours at 4°C. Tissue slices were stored in 0.01% sodium azide in PBS and washed tree times in PBS prior to the staining. Then, tissue slices were blocked for 1 hour in 7% normal goat serum (NGS).

For microglial activation (Mac-1 staining), sectioned tissues are immunostained with rat CD11b primary antibody (AbD Serotec, Raleigh, NC) diluted 1:200 in 7% NGS overnight at 4°C. Samples were incubated with goat anti-rat secondary antibody Alexa Fluor 594 (Invitrogen Corporation, Carlsbad, CA), diluted 1:200 in 7% NGS for 45 minutes at room temperature.

For astrocytosis, tissue sections were permeabilized with 1% Triton X-100 in 5% NGS (normal goat serum) in PBS for 10 minutes and blocked for 1 hour with 5% NGS then incubated with rabbit antiglial fibrillary acidic protein primary Abs diluted 1:1000 in 5% NGS for 18 hours at 4°C. Samples were incubated with goat anti-rabbit 488 (Molecular Probes), diluted 1:200 for 45 minutes at room temperature. The slices were mounted in Aquamount. Immunoreactivity was evaluated by fluorescent analysis. Fluorescence intensity was calculated using ImageJ software (National Institute of Health; NIH). Area was measured as the function of CD11b expression level using ImageJ software.

**Table 13: Immunohistochemical analysis for microglial activation and astrocytosis in the nigrostrial system.**

| Treatment groups | Intensity of fluorescence (pixels) | |
|---|---|---|
| | Micriglial activation (Mac-1 staining) | Astrocytosis (GFAP staining) |
| Naive mice (saline injected) | 28.3 ± 13.0 | 209.3 ± 3.8 |
| MPTP intoxicated | 4059.9 ± 1413.0 | 316.9 ± 4.6 |
| MPTP intoxicated and then treated with catalase polyion complex loaded into BMM | 70.9 ± 36.8 | 95.3 ± 8.3 |

The data presented in Table 13 clearly indicates that MPTP injections cause significant inflammation within the substantia nigra pars compacta and resulted in micriglial activation and astrocytosis. In contrast, treatment of MPTP-injected mice with catalase-loaded monocytes prevented neuroinflammation to the level in healthy animals (Table 13).

### Neuroprotection effect of monocytes loaded with catalase polyion complex against MPTP-induced dopaminergic neuronal loss in mice.

To quantitatively and non-invasively assess for the effect of catalase-augmented neuroprotection in the substantia nigra and striatum caused to the progression of PD in MPTP-intoxicated mice, novel neuroimaging readouts evaluating neuronal N-acetyl aspartate (NAA) levels were obtained by magnetic resonance spectroscopic imaging (MRSI).

For this purpose, first, mice were pre-scanned before MPTP injections. Then, half of the mice were injected with BMM loaded with catalase polyion complex (25 mln BMM/100 µl/mouse). MPTP-treated mice injected with PBS served as controls for maximum neurodegeneration. The brain neuronal metabolite N-acetyl aspartate (NAA) in the SNpc and stratum were assessed by MRSI on day seven after the treatment. MRI and MRSI were acquired on a Bruker Avance 7T/21 cm system operating at 300.41 MHz using actively decoupled 72 mm volume coil transmit and a laboratory built 1.25 x 1.5 cm receive surface coil. MR images were acquired with a 20 mm FOV, 25 contiguous 0.5 mm thick slices, interleaved slice order, 128 x 128 matrix, eight echoes, 12 ms echo spacing, refocused with CPMG phase cycled RF refocusing pulses to form eight images used for T2 mapping and co-registration with histology. Spectroscopic images were obtained using a numerically optimized binomial excitation refocused using three orthogonal slice selective refocusing pulses (Binomial Excitation with Volume selective Refocusing, BEVR). Spectroscopic images were obtained by selecting an 8 x 4.2 x 1.5 mm volume of interest, using 24 x 24 spatial encoding over a 20 mm field of view (FOV) with four averages in the slice containing the SNpc yielding a nominal voxel size of 1 µl. The total acquisition time is 80 min. MRSI processing. Spectroscopic images were Fourier transformed in the phase encoding dimensions and reformatted using Matlab (Mathworks Inc, Nantick, MA). Spectra were fit using AMARES in the jMRUI package. Model parameters and constraints were generated using spectra from phantoms.

Unsuppressed water spectroscopic images are obtained with identical metabolite spectra parameters except for: TR = 1 s, NA = 1 and receiver gain = 1000. The unsuppressed water is used as an internal standard for each voxel in order to quantitate metabolite concentrations from the water suppressed MRSI data. A technologist, blinded to the data source, fits the data. Calibration of the ratio of metabolite to water signal amplitude at the respective receiver gains was measured in phantom studies. Calculations were performed using Matlab (The Mathworks Inc, Nantick, MA) and metabolite concentrations were output as ASCII (for database development) and binary (for MRI overlay) metabolite maps.

As is seen in Figure 19, MPTP injections caused significant loss of NAA in SNpc and stratum of control mice. In contrast, there was no reduction in NAA levels in MPTP-intoxicated mice treated with polypeptide-polyion complex-loaded BMM. These results indicated that loaded cells can reach the damaged region of the brain in meaningful levels and release active catalase to cause subsequent neuroprotective effects in a murine PD model.

### EXAMPLE 10

### Accumulation of catalase polyion complex in various types of cell carriers.

Beside BMM, other cell carriers, such as dendritic cells (DC) or T lymphocytes, which were also demonstrated to infiltrate the brain under inflammatory conditions, can be used for catalase polyion complex delivery. The loading experiments were performed similar to those with BMM. Briefly, DC or T-lymphocytes were seeded into 96-well plates at a density of 1x106 cells/well and incubated with Alexa Fluor 594-labeled catalase polyion complex (+/- charge ratio (Z) = 1) for various time intervals. Then, the cells were washed and disrupted with 1% Triton X100. The amount of fluorescence accumulated in the BMM was assayed and normalized for the amount of cells (Table 14).

**Table 14: Accumulation of catalase polyion complex in BMM, DC and T-lymphocytes.**

| Time (min) | Amount of loaded catalase (µg/mg prot) | | |
|---|---|---|---|
| | BMM | DC | T-lymphocytes |
| 5 | 104.75 ± 25.1 | 419.78 ± 25.1 | 118.96 ± 34.8 |
| 15 | 277.45 ± 20.3 | 986.22 ± 108.98 | 279.93 ± 31.8 |
| 30 | 455.26 ± 45.1 | 1766.52 ± 206.07 | 411.89 ± 71.38 |
| 45 | 502.24 ± 84.7 | 1824.12 ± 132.75 | 271.58 ± 8.36 |
| 60 | 513.4 ± 25.1 | 1798.14 ± 78.91 | 564.31 ± 94.17 |
| 90 | 630.2 ± 46.5 | 2796.92 ± 62.56 | 542.19 ± 35.95 |

It is demonstrated that, similar to BMM, both cells rapidly (in 1 hour) take up a significant amount of catalase nanoparticles (112 µg, 21 µg, and 30 µg per 10⁶ DC, T lymphocytes, and BMM, respectively). This allows using various cell carrier systems to ensure successful brain delivery of catalase polyion complex.

### EXAMPLE 11

### Cross-linking of catalase polyion complex

To stabilize the complex various linker agents cross-linking block copolymer with the protein were used.

### Glutaraldehyde

To obtain catalase polyion complex, 0.5 ml solution of catalase (0.5 mg/ml) in 60 mM phosphate buffer, pH=7.4, was mixed with 0.5 ml solution of block copolymer (0.25 mg/ml) in the same buffer. Then, 4 µl (100 x excess (an amount of NH₂-groups) of glutaraldehyde (Fluka, # 49632, 25% water solution) was added to the mixture at vigorous stirring. The mixture was incubated for two hours at room temperature. Then, 7.5 µl of sodium borohydride solution (5x10⁻²M) in 1 M NaOH was added by two portions 20 minutes apart. The mixture was further incubated for one hour at RT, and purified by gel-filtration on Sephadex G25 column.

### N-ethyl-N'-(3-dimethylaminopropyl)-carbodiimide (EDC)

Catalase polyion complex was obtained as described above. Then, 1.5 mg EDC (30 x excess (an amount of COO-groups) was added to the mixture at vigorous stirring. The mixture was incubated for two hours at room temperature. Following incubation, the mixture was further purified by gel-filtration on Sephadex G25 column.

### Bis-(sulfosuccinimidyl)suberate sodium salt (BS3)

Catalase polyion complex was obtained as described above. Then, 2 mg BS3 (7 x excess (an amount of Lysine groups) was added to the mixture at vigorous stirring. The mixture was incubated for three hours at room temperature. Following incubation, the mixture was further purified by gel-filtration on Sephadex G25 column.

A cross-linking of catalase polyion complexes was confirmed by Western blot. Samples were subjected to gel electrophoresis in polyacrylamide gel (10%) under denaturing conditions (with SDS) that destroyed non-linked complex. Then, gels were blotted and protein bands were visualized with primary antibody to catalase (abcam, ab1877). Figure 20 provides images of catalase/polyion complexes cross-linked using various linkers. Lane 1: latter; lane 2: catalase alone; line 3: catalase polyion complex linked with EDC; line 4: catalase polyion complex linked with GA; line 5: catalase polyion complex linked with BS3.

As is seen in Figure 20, complete conjugation was achieved with GA resulting in the absence of catalase band due to the large complexes that did not enter the gel (line 4, no band of catalase). Using EDC as a linker agent (line 3) also resulted in cross-linking although complete conjugation was not achieved under these conditions as some band of free catalase is present. Linking with BS3 (line 5) produced smaller complexes that entered the gel, although with retardation compared the free catalase band (line 2).

### Cross-linking of superoxide dismutase (SOD) polyion complex

Similar cross-linking complexes were obtained with SOD and the block copolymer.

### GA

To obtain SOD polyion complex, 0.5 ml solution of SOD (1 mg/ml) in 60 mM phosphate buffer, pH=7.4, was mixed with 0.5 ml solution of block copolymer (0.25 mg/ml) in the same buffer. Then, 10 µl (100 x excess (an amount of NH₂-groups) of GA was added to the mixture at vigorous stirring. The mixture was incubated for two hours at room temperature. Then, 5 µl of sodium borohydride solution (5x10-2 M) in 1 M NaOH was added by two portions 20 minutes apart. The mixture was further incubated for one hour at room temperature, and purified by gel-filtration on Sephadex G25 column.

### EDC

SOD polyion complex was obtained as described above. Then, 1.5 mg EDC (12 x excess (an amount of COO-groups) was added to the mixture at vigorous stirring. The mixture was incubated for two hours at room temperature. Following incubation, the mixture was further purified by gel-filtration on Sephadex G25 column.

### BS3

SOD polyion complex was obtained as described above. Then, 1.7 mg BS3 (4.5 x excess (an amount of Lysine groups) was added to the mixture at vigorous stirring. The mixture was incubated for three hours at room temperature. Following incubation, the mixture was further purified by gel-filtration on Sephadex G25 column.

A cross-linking of SOD polyion complexes was confirmed by Western blot. Samples were subjected to gel electrophoresis in polyacrylamide gel (10%) under denaturing conditions (with SDS) that destroyed non-linked complex. Then, gels were blotted and protein bands were visualized with primary antibody to SOD (Calbiochaem, # 574597). Figure 21 provides images of SOD/polyion complexes cross-linked using various linkers. Lane 1: latter; lane 2: SOD alone; line 3: non-linked SOD polyion line 4: SOD polyion complex linked with EDC; line 5: SOD polyion complex linked with GA; line 6: SOD polyion complex linked with BS3.

As is seen in Figure 21, cross-linking with EDC (line 4) did not accomplish complete conjugation under these specific conditions as some band of free SOD is present. In contrast, complete conjugation was achieved with GA (line 5) and BS3 (line 6) resulting in the absence of SOD band due to the obtaining large complexes that did not enter the gel.

### Cross-linking of catalase/SOD polyion complex

Overall, to obtain mixed catalase/SOD polyion complex, first, catalase and SOD were mixed at pH 6.8 (catalase is charged negatively (PI 7.28) and SOD is charged positively (PI 6.32) at this pH). Then, the block copolymer was added, and various linkers were used to conjugate the block copolymer with the proteins similar to the synthesis described above.

### GA

To obtain catalase/SOD polyion complex, 1 mg catalase and 1.33 mg SOD were dissolved in 60 mM phosphate buffer, pH=6.8. Then, 1.3 mg the block copolymer was added to the mixture and incubated for 10 minutes at room temperature. 5 µl (9 x excess (an amount of NH₂-groups) of GA was added to the mixture at vigorous stirring. The mixture was incubated overnight (8 hours) at 4°C. Then, 6.5 µl of sodium borohydride solution (5x10⁻² M) in 1 M NaOH was added by two portions 20 minutes apart. The mixture was further incubated for one hour at room temperature, and purified by gel-filtration on Sephadex G25 column.

### EDC

Catalase/SOD polyion complex was obtained as described above. Then, 10 mg EDC (20 x excess (an amount of COO- groups) was added to the mixture at vigorous stirring. The mixture was incubated overnight (8 hours) at 4°C. Following incubation, the mixture was further purified by gel-filtration on Sephadex G25 column.

### BS3

Catalase/SOD polyion complex was obtained as described above. Then, 8.6 mg BS3 (10 x excess (an amount of Lysine groups) was added to the mixture at vigorous stirring. The mixture was incubated for three hours at room temperature. Following incubation, the mixture was further purified by gel-filtration on Sephadex G25 column.

### EDC-sulfo-NHS

To stabilize intermediate EDC complex, sulfo-N-hydroxysuccineimide (sulfo-NHS) was used. For this purpose, catalase/SOD polyion complex was obtained as described above. Then, 10 mg EDC (20 x excess (an amount of COO- groups) was added to the mixture at vigorous stirring. Following addition of EDC, 2 mg sulfo-NHS was added, and the reaction mixture was incubated for 3 hours at room temperature. Following incubation, the mixture was further purified by gel-filtration on Sephadex G25 column.

A cross-linking of catalase/SOD polyion complexes was confirmed by Western blot. Samples were subjected to gel electrophoresis in polyacrylamide gel (10%) under denaturing conditions (with SDS). Then, gels were blotted and protein bands were visualized with primary antibody to catalase and SOD separately. Figure 22A provides images of catalase/SOD/polyion complexes cross-linked using various linkers labeled with ab to catalase. Lane 1: non-linked catalase/SOD polyion complex; catalase/SOD polyion complexes linked with GA (EDC; line 5: SOD polyion complex linked with GA (lane 2); EDC (line 3); BS3 (line 4); EDC-S-NHS (line 5). As is seen in the Figure, a complete conjugation was achieved with GA (line 2); cross-linking with EDC (line 3) resulted in incomplete conjugation (some band of free catalase is present). Using BS3 linker (line 4) resulted in complexes that were able to enter the gel, although with retardation compared to non-linked catalase/SOD polyion complex. Stabilization of intermediate EDC complex with sulfo-N-hydroxysuccineimide (line 5) resulted in significantly better cross-linking compared to EDC alone (line 3).

Figure 22B provides images of catalase/SOD/polyion complexes cross-linked using various linkers labeled with ab to SOD. Lane 1: non-linked catalase/SOD polyion complex; catalase/SOD polyion complexes linked with GA (EDC; line 5: SOD polyion complex linked with GA (lane 2); EDC (line 3); BS3 (line 4); EDC-S-NHS (line 5).

The results confirmed data from the gel stained with ab to catalase. A complete conjugation was achieved with GA (line 2); cross-linking with EDC (line 3) resulted in non-complete conjugation (significant staining of free SOD is present). Using BS3 linker (line 4) and sulfo-N-hydroxysuccineimide along with EDC (line 5) resulted in almost complete conjugation.

### EXAMPLE 12

### Visualization of BMM biodistribution in MPTP-intoxicated mice

Prior to the experiment, BALB/C female mice were anesthetized with pentobarbital i.p. injections at the dose of 30-40mg/kg body weight, shaved and depilated (to reduce fluorescence blocking by hair). The mice were kept on liquid diet for 72 hours (to eliminate autofluorescence in stomach and intestine from solid food). Mice were administered at 18 mg freebase MPTP/kg body weight delivered in PBS by 4 intraperitoneal injections given every two hours (MPTP (Sigma Chemical Co., St. Louis, MO)). 18 hours later the mice were tail vein-injected with Li-COR labeled BMM (50 mln/mouse) loaded with catalase polyion complex. Then, the mice were anesthetized with a 1.5% isoflurane mixture with 66% nitrous oxide and the remainder oxygen and placed into imaging camera. The biodistribution of labeled BMM loaded with catalase polyion complex was determined by measuring the in vivo fluorescence of Li-COR as detected by an IVIS 200 Series Imaging Gas Anasthesia System. Li-COR-labeled BMM loaded with catalase polyion complex started to accumulate in the brain 2 hours after IV injection, peaked at 4-7 hours post-injection, and remained elevated for at least 48 hours post-injection (Figure 23). These data indicate that peripherally administered BMM loaded with catalase polyion complex were able to reach and accumulate in the brain of MPTP-intoxicated mice in significant quantities.

While certain of the preferred embodiments of the present invention have been described and specifically exemplified above, it is not intended that the invention be limited to such embodiments.

## Claims

1. A pharmaceutical composition for use in treating a neurological disorder of the central nervous system, said composition comprising:
a) at least one complex comprising a therapeutic polypeptide and a synthetic polymer, wherein said synthetic polymer is a block copolymer comprising at least one water soluble, nonionic segment and at least one polyion segment, wherein said complex self-assembles into nanoparticles having a core-shell morphology wherein the core comprises the therapeutic polypeptide and the polyion segment of the block copolymer, and wherein said polyion segment comprises at least one charge opposite to the charge of the therapeutic polypeptide, and
b) at least one pharmaceutically acceptable carrier.

2. The composition for use according to claim 1 wherein said at least one complex is comprised within an isolated cell.

3. The composition for use according to claim 1 or claim 2, wherein said synthetic polymer is negatively charged and said therapeutic polypeptide has a net positive charge at pH 7.4 or wherein said synthetic polymer is positively charged and said therapeutic polypeptide has a net negative charge at pH 7.4.

4. The composition for use according to claim 3, wherein said polyion segment is selected from the group consisting of polyalkyleneimine, polylysine, polyarginine, polyaspartic acid, polyglutamic acid, polyacrylic acid, polyalkylene acrylic, and their copolymers.

5. The composition for use according to claim 1 or claim 2, wherein said therapeutic polypeptide is selected from the group consisting of an enzyme, an antibody, a hormone, and a growth factor.

6. The composition for use according to claim 1 or claim 2, wherein said therapeutic polypeptide exhibits central nervous system therapeutic activity.

7. The composition for use according to claim 1 or claim 2, wherein said therapeutic polypeptide is selected from the group consisting of endocrine factors, growth factors, hypothalamic releasing factors, neurotrophic factors, paracrine factors, neurotransmitter polypeptides, antibodies, antibody fragments, cytokines, endorphins, polypeptide antagonists, agonists for a receptor expressed by a CNS cell, lysosomal storage disease polypeptides, and antiapoptotic proteins.

8. The composition for use according to claim 1 or claim 2, wherein said therapeutic polypeptide is selected from the group consisting of catalase, superoxide dismutase, and glutathioneperoxidase.

9. The composition for use according to claim 1 or claim 2, wherein said therapeutic polypeptide is selected from the group consisting of butyrylcholinesterase, acetylcholinesterase, cholinesterase reactivators, scavengers of organophosphate, and carbamate inhibitors.

10. The composition for use according to claim 2, wherein said cell is isolated from the patient to be treated.

11. The composition for use according to claim 2 wherein said isolated cell is an immune cell.

12. The composition for use according to claim 11, wherein said immune cell is selected from the group consisting of bone marrow monocytes, monocytes, macrophages, bone marrow derived monocytes, dendritic cells, lymphocytes, T-cells, neutrophils, eosinophils, and basophils.

13. The composition for use according to claim 1 or claim 2, wherein said neurological disorder is a neuroinflammatory disease or disorder and said therapeutic polypeptide is selected from the group consisting of catalase, superoxide dismutase, and glutathione peroxidase.

14. The composition for use according to claim 13, wherein said neuroinflammatory disease or disorder is Parkinson's disease.

15. The composition for use according to claim 1 or claim 2, wherein said water-soluble, nonionic segment is poly(ethylene oxide).

16. The pharmaceutical composition for use according to claim 1 or claim 2, wherein said composition is formulated for intravenous administration.

## Patentansprüche

1. Pharmazeutische Zusammensetzung zur Verwendung zur Behandlung einer neurologischen Störung des Zentralnervensystems, wobei die Zusammensetzung Folgendes umfasst:
a) zumindest einen Komplex, der ein therapeutisches Polypeptid und ein synthetisches Polymer umfasst, worin das synthetische Polymer ein Blockcopolymer ist, das zumindest ein wasserlösliches, nichtionisches Segment und zumindest ein Polyionensegment umfasst, worin der Komplex sich selbst zu Nanopartikeln mit einer Kern-Schale-Morphologie assembliert, worin der Kern das therapeutische Polypeptid und das Polyionensegment des Blockcopolymers umfasst, und worin das Polyionensegment zumindest eine der Ladung des therapeutischen Polypeptids entgegengesetzte Ladung umfasst, und
b) zumindest einen pharmazeutisch annehmbaren Träger.

2. Zusammensetzung zur Verwendung nach Anspruch 1, worin der zumindest eine Komplex in einer isolierten Zelle umfasst ist.

3. Zusammensetzung zur Verwendung nach Anspruch 1 oder Anspruch 2, worin das synthetische Polymer negativ geladen ist und das therapeutische Polypeptid eine positive Nettoladung bei pH 7,4 aufweist oder worin das synthetische Polymer positiv geladen ist und das therapeutische Polypeptid eine negative Nettoladung bei pH 7,4 aufweist.

4. Zusammensetzung zur Verwendung nach Anspruch 3, worin das Polyionensegment aus der aus Polyalkylenimin, Polylysin, Polyarginin, Polyasparaginsäure, Polyglutaminsäure, Polyacrylsäure, Polyalkylenacrylsäure und deren Copolymeren bestehenden Gruppe ausgewählt ist.

5. Zusammensetzung zur Verwendung nach Anspruch 1 oder Anspruch 2, worin das therapeutische Polypeptid aus der aus einem Enzym, einem Antikörper, einem Hormon und einem Wachstumsfaktor bestehenden Gruppe ausgewählt ist.

6. Zusammensetzung zur Verwendung nach Anspruch 1 oder Anspruch 2, worin das therapeutische Polypeptid therapeutische Aktivität am Zentralnervensystem ausübt.

7. Zusammensetzung zur Verwendung nach Anspruch 1 oder Anspruch 2, worin das therapeutische Polypeptid aus der aus endokrinen Faktoren, Wachstumsfaktoren, Hypothalamusfreisetzungsfaktoren, neurotrophen Faktoren, parakrinen Faktoren, Neutrotransmitterpolypeptiden, Antikörpern, Antikörperfragmenten, Zytokinen, Endorphinen, Polypeptidantagonisten, Agonisten für einen durch eine ZNS-Zelle exprimierten Rezeptor, Polypeptiden der lysosomalen Speicherkrankheit und antiapoptotischen Proteinen bestehenden Gruppe ausgewählt ist.

8. Zusammensetzung zur Verwendung nach Anspruch 1 oder Anspruch 2, worin das therapeutische Polypeptid aus der aus Catalase, Superoxiddismutase und Glutathionperoxidase bestehenden Gruppe ausgewählt ist.

9. Zusammensetzung zur Verwendung nach Anspruch 1 oder Anspruch 2, worin das therapeutische Polypeptid aus der aus Butyrylcholinesterase, Acetylcholinesterase, Cholinesterasereaktivatoren, Organophosphatfängern und Carbamatinhibitoren bestehenden Gruppe ausgewählt ist.

10. Zusammensetzung zur Verwendung nach Anspruch 2, worin die Zelle von dem zu behandelnden Patienten isoliert ist.

11. Zusammensetzung zur Verwendung nach Anspruch 2, worin die isolierte Zelle eine Immunzelle ist.

12. Zusammensetzung zur Verwendung nach Anspruch 11, worin die Immunzelle aus der aus Knochenmarksmonozyten, Monozyten, Makrophagen, vom Knochenmark stammenden Monozyten, dendritischen Zellen, Lymphozyten, T-Zellen, Neutrophilen, Eosinophilen und Basophilen bestehenden Gruppe ausgewählt ist.

13. Zusammensetzung zur Verwendung nach Anspruch 1 oder Anspruch 2, worin die neurologische Störung eine Nervenentzündungskrankheit oder -störung ist und das therapeutische Polypeptid aus der aus Catalase, Superoxiddismutase und Glutathionperoxidase bestehenden Gruppe ausgewählt ist.

14. Zusammensetzung zur Verwendung nach Anspruch 13, worin die Nervenentzündungskrankheit oder -störung die Parkinson-Krankheit ist.

15. Zusammensetzung zur Verwendung nach Anspruch 1 oder Anspruch 2, worin das wasserlösliche, nichtionische Segment Poly(ethylenoxid) ist.

16. Pharmazeutische Zusammensetzung zur Verwendung nach Anspruch 1 oder Anspruch 2, worin die Zusammensetzung zur intravenösen Verabreichung formuliert ist.

## Revendications

1. Composition pharmaceutique pour utilisation dans le traitement d'un trouble neurologique du système nerveux central, ladite composition comprenant:
a) au moins un complexe comprenant un polypeptide thérapeutique et un polymère synthétique, dans lequel ledit polymère synthétique est un copolymère séquencé comprenant au moins un segment non ionique hydrosoluble et au moins un segment polyionique, où ledit complexe s'auto-assemble en nanoparticules ayant une morphologie coeur-enveloppe où le coeur comprend le polypeptide thérapeutique et le segment polyionique du copolymère séquencé, et où ledit segment polyionique comprend au moins une charge opposée à la charge du polypeptide thérapeutique, et
b) au moins un véhicule pharmaceutiquement acceptable.

2. Composition pour utilisation selon la revendication 1 dans laquelle ledit au moins un complexe est contenu dans une cellule isolée.

3. Composition pour utilisation selon la revendication 1 ou la revendication 2, dans laquelle ledit polymère synthétique est chargé négativement et ledit polypeptide thérapeutique a une charge nette positive à pH 7,4 ou dans laquelle ledit polymère synthétique est chargé positivement et ledit polypeptide thérapeutique a une charge nette négative à pH 7,4.

4. Composition pour utilisation selon la revendication 3, dans laquelle ledit segment polyionique est sélectionné dans le groupe constitué par une polyalkylèneimine, une polylysine, une polyarginine, un polyacide aspartique, un polyacide glutamique, un polyacide acrylique, un polyalkylène-acrylique, et leurs copolymères.

5. Composition pour utilisation selon la revendication 1 ou la revendication 2, dans laquelle ledit polypeptide thérapeutique est sélectionné dans le groupe constitué par une enzyme, un anticorps, une hormone, et un facteur de croissance.

6. Composition pour utilisation selon la revendication 1 ou la revendication 2, dans laquelle ledit polypeptide thérapeutique possède une activité thérapeutique sur le système nerveux central.

7. Composition pour utilisation selon la revendication 1 ou la revendication 2, dans laquelle ledit polypeptide thérapeutique est sélectionné dans le groupe constitué par les facteurs endocrines, les facteurs de croissance, les facteurs de libération hypothalamique, les facteurs neurotrophiques, les facteurs paracrines, les polypeptides neurotransmetteurs, les anticorps, les fragments d'anticorps, les cytokines, les endorphines, les antagonistes de polypeptides, les agonistes de récepteur exprimé par une cellule du SNC, les polypeptides de maladies du stockage lysosomal, et les protéines antiapoptotiques.

8. Composition pour utilisation selon la revendication 1 ou la revendication 2, dans laquelle ledit polypeptide thérapeutique est sélectionné dans le groupe constitué par la catalase, la superoxyde dismutase, et la glutathion peroxydase.

9. Composition pour utilisation selon la revendication 1 ou la revendication 2, dans laquelle ledit polypeptide thérapeutique est sélectionné dans le groupe constitué par la butyrylcholinestérase, l'acétylcholinestérase, les réactivateurs des cholinestérases, les pièges à organophosphates, et les inhibiteurs de carbamates.

10. Composition pour utilisation selon la revendication 2, dans laquelle ladite cellule est isolée à partir du patient à traiter.

11. Composition pour utilisation selon la revendication 2 dans laquelle ladite cellule isolée est une cellule immunitaire.

12. Composition pour utilisation selon la revendication 11, dans laquelle ladite cellule immunitaire est sélectionnée dans le groupe constitué par les monocytes de moelle osseuse, les monocytes, les macrophages, les monocytes dérivés de moelle osseuse, les cellules dendritiques, les lymphocytes, les lymphocytes T, les neutrophiles, les éosinophiles, et les basophiles.

13. Composition pour utilisation selon la revendication 1 ou la revendication 2, dans laquelle ledit trouble neurologique est un trouble ou une maladie neuroinflammatoire et ledit polypeptide thérapeutique est sélectionné dans le groupe constitué par la catalase, la superoxyde dismutase, et la glutathion peroxydase.

14. Composition pour utilisation selon la revendication 13, dans laquelle ledit trouble ou ladite maladie neuroinflammatoire est la maladie de Parkinson.

15. Composition pour utilisation selon la revendication 1 ou la revendication 2, dans laquelle ledit segment non ionique hydrosoluble est un poly(oxyde d'éthylène).

16. Composition pharmaceutique pour utilisation selon la revendication 1 ou la revendication 2, ladite composition étant formulée pour une administration intraveineuse.
